# EUROPEAN PATENT APPLICATION

(11) **EP 1 782 832 A1**
(43) Date of publication of application: **09.05.2007**
(21) Application number: 05777050.5
(22) Date of filing: 25.08.2005
(51) Int. Cl.: A61K 45/06, A61K 31/4439, A61K 31/455, A61P 1/18, A61P 3/10, A61K 31/4985, A61K 31/40

(54) **REMEDY FOR DIABETES**

(30) Priority: 26.08.2004 JP 2004246620
(71) Applicant: Takeda Pharmaceutical Company Limited, Osaka-shi, Osaka 5408645 (JP)
(72) Inventor: SUZUKI, Nobuhiro Takeda Pharmaceutical Comp. Ltd, Tsukuba-shi, Ibaraki 300-4293 (JP); MORITOH, Yusuke Takeda Pharmaceutical Comp. Ltd, Osaka-s-shi, Osaka 532-8686 (JP); WATANABE, Masanori Takeda Pharmaceutical Comp. Ltd, Osaka-s-shi, Osaka 532-8686 (JP)
(74) Representative: Lewin, John Harvey
(86) International application number: PCT/JP2005/015956
(87) International publication number: WO 2006/022428

(57) **Abstract**

The present invention provides an agent for increasing the pancreatic insulin content useful for the treatment of diabetes and the like. In one embodiment, the present invention provides an agent for increasing the pancreatic insulin content, which contains a blood glucose lowering drug that does not stimulate insulin secretion, and a dipeptidyl-peptidase IV inhibitor in combination.

## Description

### Technical Field

The present invention relates to an agent for increasing a pancreatic insulin content, which is useful for the treatment of diabetes and the like.

### Background Art

It is known that the amount of pancreatic β cells remarkably decreases not only in type 1 but also type 2 diabetes (fasting blood glucose: FPG>126 mg/dL), and the decrease in the amount of pancreatic β cells has also been acknowledged in IFG (Impaired Fasting Glucose) (110<FPG<125 mg/dL) wherein the fasting blood glucose is somewhat above the normal level. While the amount of pancreatic β cells is histologically quantified as the amount of insulin-positive β cells, a pancreatic insulin content is considered to reflect the amount of pancreatic β cells and an insulin content of individual β cell. Accordingly, for the basic treatment of diabetes, therefore, a pharmaceutical agent positively increasing a pancreatic insulin content that reflect both the amount of pancreatic β cells and the insulin content of individual β cell is considered to be effective.

The amount of pancreatic β cells is controlled by regeneration, replication and cell death due to apoptosis of β cells and, in diabetes, pancreatic βcells are exhausted and the pancreatic β cell death is finally promoted. Accordingly, a pharmaceutical agent that increases the pancreatic insulin content and has a pancreas protection activity such as suppression of pancreatic exhaustion, pancreatic β cell death, and the like is considered to be extremely effective for the treatment of diabetes.

Dipeptidyl-peptidase IV (hereinafter sometimes to be abbreviated as DPP-IV) inhibitors are known to be useful as a therapeutic drug for diabetes and the like (e.g., WO02/062764 and WO2004/014860).

In addition, DPP-IV inhibitors are known to be usable in combination with anti-diabetes compounds (e.g., WO01/52825 and U.S. patent application publication No. 2003/0166578).

However, no report has documented that a combination of a DPP-IV inhibitor and a blood glucose lowering drug that does not stimulate insulin secretion is useful as an agent for increasing the pancreatic insulin content.

### Disclosure of the Invention

It is an object of the present invention to provide an agent for increasing the pancreatic insulin content, which is useful for the treatment of diabetes and the like and free of side effects.

The present inventors have first found that a combination of a DPP-IV inhibitor and a blood glucose lowering drug that does not stimulate insulin secretion affords unexpectedly superior effects on increasing pancreatic insulin content, which resulted in the completion of the present invention.

Accordingly, the present invention relates to
1) an agent for increasing a pancreatic insulin content, which comprises a blood glucose lowering drug that does not stimulate insulin secretion, and a dipeptidyl-peptidase IV inhibitor in combination;
2) the agent of the aforementioned 1), wherein the blood glucose lowering drug that does not stimulate insulin secretion is an insulin sensitizer;
3) the agent of the aforementioned 2), wherein the insulin sensitizer is pioglitazone or a salt thereof;
4) a method of increasing a pancreatic insulin content of a mammal, which comprises administering a blood glucose lowering drug that does not stimulate insulin secretion, and a dipeptidyl-peptidase IV inhibitor to the mammal;
5) use of a blood glucose lowering drug that does not stimulate insulin secretion, and a dipeptidyl-peptidase IV inhibitor for the production of an agent for increasing a pancreatic insulin content;
6) an agent that enhances pancreas protection activity of a dipeptidyl-peptidase IV inhibitor, which comprises a blood glucose lowering drug that does not stimulate insulin secretion;
7) the agent of the aforementioned 6), wherein the blood glucose lowering drug that does not stimulate insulin secretion is an insulin sensitizer;
8) the agent of the aforementioned 7), wherein the insulin sensitizer is pioglitazone or a salt thereof;
9) a method of enhancing a pancreas protection activity of a dipeptidyl-peptidase IV inhibitor in a mammal, which comprises administering a blood glucose lowering drug that does not stimulate insulin secretion to the mammal;
10) use of a blood glucose lowering drug that does not stimulate insulin secretion for the production of an agent that enhances pancreas protection activity of a dipeptidyl-peptidase IV inhibitor;
11) an agent that enhances pancreas protection activity of a blood glucose lowering drug that does not stimulate insulin secretion, which comprises a dipeptidyl-peptidase IV inhibitor;
12) the agent of the aforementioned 11), wherein the blood glucose lowering drug that does not stimulate insulin secretion is an insulin sensitizer;
13) the agent of the aforementioned 12), wherein the insulin sensitizer is pioglitazone or a salt thereof;
14) a method of enhancing a pancreas protection activity of a blood glucose lowering drug that does not stimulate insulin secretion in a mammal, which comprises administering a dipeptidyl-peptidase IV inhibitor to the mammal;
15) use of a dipeptidyl-peptidase IV inhibitor for the production of an agent that enhances pancreas protection activity of a blood glucose lowering drug that does not stimulate insulin secretion;
16) a method of synergistically protecting the pancreas of a mammal as compared to a single administration of a blood glucose lowering drug that does not stimulate insulin secretion, or a dipeptidyl-peptidase IV inhibitor, which comprises administering a blood glucose lowering drug that does not stimulate insulin secretion, and a dipeptidyl-peptidase IV inhibitor to the mammal; and the like.

The agent for increasing a pancreatic insulin content of the present invention affords a superior effects in increasing pancreatic insulin content, and is useful for the treatment of diabetes and the like.

The agent for increasing a pancreatic insulin content and the agent that enhances pancreas protection activity of the present invention show a superior hypoglycemic action and a superior blood insulin level increasing action in diabetic patients, and can further suppress progression from diabetes to diabetic complications (e.g., diabetic neuropathy, diabetic nephropathy, diabetic retinopathy, arteriosclerosis).

Moreover, the agent for increasing a pancreatic insulin content and the agent that enhances pancreas protection activity of the present invention suppress glucose toxicity due to diabetes, lipotoxicity, and pancreatic exhaustion due to an oxidative stress or an endoplasmic reticulum stress and the like, and can maintain glucose-dependent insulin secretary ability, which is an important function of pancreatic β cells.

Moreover, the agent for increasing a pancreatic insulin content and the agent that enhances pancreas protection activity of the present invention can suppress pancreatic β cell death due to diabetes and can promote regeneration or replication of pancreatic β cells.

Furthermore, although the agent for increasing a pancreatic insulin content and the agent that enhances pancreas protection activity of the present invention initiate promotion of a glucose-dependent insulin secretion, they are free of side effects associated with insulin preparations (e.g., vascular complications, hypoglycemia) and insulin secretion type blood glucose lowering drugs acting on a sulfonylurea receptor (e.g., pancreatic exhaustion, hypoglycemia). Therefore, the agent for increasing a pancreatic insulin content and the agent that enhances pancreas protection activity of the present invention can be safely administered for a long time to patients affected with diabetes and the like.

### Best Mode for Embodying the Invention

In the present specification, the blood glucose lowering drug that does not stimulate insulin secretion means a compound that lowers blood glucose by an action mechanism other than the insulin secretion from pancreatic β cells. While the compound may be peptidic or nonpeptidic, a nonpeptidic one is preferable.

In addition, the blood glucose lowering drug that does not stimulate insulin secretion may be in different forms before and after administration into the living body, as long as the blood glucose lowering activity without stimulation of insulin secretion is maintained. In other words, the blood glucose lowering drug that does not stimulate insulin secretion may be an "active metabolite" that acquires a blood glucose lowering activity without stimulation of insulin secretion after becoming a structure-modified drug by metabolism in vivo. Moreover, the blood glucose lowering drug that does not stimulate insulin secretion may be a "prodrug" that changes to an active form by reaction of enzyme, gastric acid and the like under physiological conditions in the living body.

Specific examples of the blood glucose lowering drug that does not stimulate insulin secretion, include insulin sensitizers, biguanides, somatostatin receptor agonists, 11β-hydroxysteroid dehydrogenase inhibitors, α-glucosidase inhibitors and the like. Two or more kinds thereof may be used in combination at an appropriate ratio.

Examples of the insulin sensitizer include pioglitazone and a salt thereof (preferably hydrochloride), rosiglitazone and a salt thereof (preferably maleate), Reglixane (JTT-501), Netoglitazone (MCC-555), Rivoglitazone (CS-011), FK-614, compounds described in WO99/58510 (e.g., (E)-4-[4-(5-methyl-2-phenyl-4-oxazolylmethoxy)benzyloxyimino]-4-phenylbutyric acid), compounds described in W
O01/38325, Tesaglitazar (AZ-242), Ragaglitazar (NN-622), Muraglitazar (BMS-298585), Edaglitazone (BM-13-1258), Naveglitazar (LY-818), Metaglidasen (MBX-102), LY-510929, Balaglitazone (NN-2344), T-131 and a salt thereof, THR-0921 and the like. Of these, thiazolidinedione compounds are preferable, and pioglitazone and a salt thereof (preferably hydrochloride) are more preferable.

Examples of biguanides include metformin, phenformin, buformin and a salt thereof (e.g., hydrochloride, fumarate, succinate) and the like. Of these, metformin and a salt thereof (preferably hydrochloride) are preferable.

Examples of the somatostatin receptor agonists include the compounds described in WO01/25228, WO03/42204, WO98/44921, WO98/45285, WO99/22735 and the like, and the like. Of these, somatostatin subtype 2 receptor agonists are preferable.

Examples of the 11β-hydroxysteroid dehydrogenase inhibitor include BVT-3498 and the like.

Examples of the α-glucosidase inhibitors include voglibose, acarbose, miglitol, emiglitate and the like.

The blood glucose lowering drug that does not stimulate insulin secretion, is preferably an insulin sensitizer or biguanide, more preferably an insulin sensitizer, and particularly preferably pioglitazone or a salt thereof (preferably hydrochloride).

In the present specification, a DPP-IV inhibitor means a compound that inhibits an enzyme activity of DPP-IV [classification by the International Union of Biochemistry and Molecular Biology (IUBMB): EC3.4.14.5]. The compound may be peptidic or nonpeptidic, but nonpeptidic one is preferable.

In addition, the DPP-IV inhibitor may be in different forms before and after administration into the living body, as long as the DPP-IV inhibitory activity is maintained. In other words, the DPP-IV inhibitor may be an "active metabolite" that acquires a DPP-IV inhibitory activity after becoming a structure-modified drug by metabolism in vivo. Moreover, the DPP-IV inhibitor may be a "prodrug" that changes to an active form by reaction of enzyme, gastric acid and the like under physiological conditions in the living body.

The DPP-IV inhibitory activity can be confirmed, for example, by a method utilizing the "method of Raymond et al. (Diabetes, Vol. 47, pages 1253-1258, 1998)".

Specific examples of the DPP-IV inhibitor include the following compounds (1)-(6). Two or more kinds thereof may be used in combination at an appropriate ratio.
(1) A compound described in WO02/062764, which is represented by the formula: 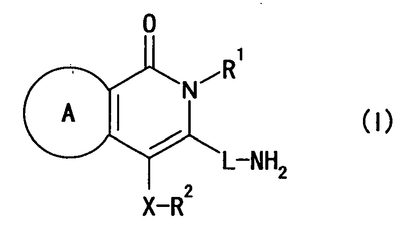 wherein
   ring A is an optionally substituted 5- to 10-membered aromatic ring,
   R¹ and R² are the same or different and each is an optionally substituted hydrocarbon group or an optionally substituted heterocyclic group,
   X is a bond, -O-, -S-, -SO-, -SO₂- or -NR³- (R³ is a hydrogen atom or an optionally substituted hydrocarbon group); and
   L is a divalent hydrocarbon group,
   or a salt thereof.

As a salt of the compound represented by the formula (I), a pharmacologically acceptable salt is preferable, and as such salt, for example, salts with inorganic base, salts with organic base, salts with inorganic acid, salts with organic acid, salts with basic or acidic amino acid and the like can be mentioned.

Preferable examples of the salts with inorganic base include alkali metal salts such as sodium salt, potassium salt and the like; alkaline earth metal salts such as calcium salt, magnesium salt and the like; aluminum salt; ammonium salt and the like.

Preferable examples of the salts with organic base include salts with trimethylamine, triethylamine, pyridine, picoline, ethanolamine, diethanolamine, triethanolamine, dicyclohexylamine, N,N-dibenzylethylenediamine and the like.

Preferable examples of the salts with inorganic acid include salts with hydrochloric acid, hydrobromic acid, nitric acid, sulfuric acid, phosphoric acid and the like.

Preferable examples of the salts with organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like.

Preferable examples of the salts with basic amino acid include salts with arginine, lysine, ornithine and the like.

Preferable examples of the salts with acidic amino acid include salts with aspartic acid, glutamic acid and the like.

The compound represented by the formula (I) may be an anhydrate or a hydrate, or a prodrug.

Preferable examples of the compound represented by the formula (I) include the following compounds.

### (Compound I-a)

A compound wherein
ring A is a benzene ring optionally having 1 or 2 substituents selected from;
1) a cyano group;
2) a C₁₋₁₀ alkyl group (preferably ethyl) or a C₂₋₁₀ alkenyl group (preferably ethenyl), each optionally substituted by a carbamoyl group or a carboxyl group;
3) an optionally substituted hydroxy group [preferably, an alkoxy group having 1 to 10 carbon atoms (preferably methoxy, isopropoxy) which optionally has 1 to 3 substituents selected from a carbamoyl group, a carboxyl group and an alkoxycarbonyl group having 2 to 5 carbon atoms (preferably methoxycarbonyl); a hydroxy group; an aralkyloxy group having 7 to 13 carbon atoms (preferably benzyloxy)] [more preferably carbamoylmethoxy];
4) an acyl group [preferably C₁₋₆ alkyl-carbonyl (preferably acetyl), carbamoyl, mono- or di-(C₁₋₆ alkyl optionally having 1 to 3 substituents selected from halogen atom and C₁₋₆ alkoxy-carbonyl)-carbamoyl (preferably methylcarbamoyl, ethylcarbamoyl, propylcarbamoyl, dimethylcarbamoyl, trifluoroethylcarbamoyl, ethoxycarbonylmethylcarbamoyl), C₃₋₁₀ cycloalkyl-carbamoyl (preferably cyclopropylcarbamoyl), C₇₋₁₃ aralkyl-carbamoyl (preferably benzylcarbamoyl), nitrogen-containing heterocycle-carbonyl (preferably pyrrolidinylcarbonyl, piperidinocarbonyl) optionally substituted by hydroxy, C₁₋₆ alkylsulfonyl (preferably methylsulfonyl), C₁₋₆ alkylsulfinyl (preferably methylsulfinyl), carboxyl, C₁₋₆ alkoxy-carbonyl (preferably methoxycarbonyl), thiocarbamoyl];
5) an optionally substituted amino group (preferably, carbamoylamino);
6) an optionally substituted thiol group [preferably, a C₁₋₁₀ alkylthio group (preferably methylthio) optionally substituted by a carbamoyl group];
7) an optionally substituted heterocyclic group [preferably, an aromatic heterocyclic group (preferably, furyl, thienyl, oxazolyl, oxadiazolyl, thiazolyl, tetrazolyl, pyridyl, pyrrolyl, triazolyl) or a nonaromatic heterocyclic group (preferably, dioxoisoindole, 5-oxooxadiazol-3-yl, 5-oxothiadiazol-3-yl), each optionally having 1 or 2 substituents selected from a C₁₋₆ alkyl group optionally substituted by 1 to 3 halogen atoms (preferably methyl, trifluoromethyl), a carboxyl group, an alkoxycarbonyl group having 2 to 8 carbon atoms (preferably ethoxycarbonyl), a cyano group, a carbamoyl group, an amino group, a mono- or di-C₂₋₁₀ alkanoylamino group (e.g., acetylamino, isopentanoylamino), a C₁₋₁₀ alkoxy-carbonylamino group (e.g., methoxycarbonylamino), a carbamoylamino group, a mono- or di-C₁₋₁₀ alkyl-carbamoylamino group (e.g., methylcarbamoylamino, dimethylcarbamoylamino), a C₆₋₁₄ aryl-carbonylamino group (e.g., benzoylamino), a C₃₋₁₀ cycloalkyl-carbonylamino group, a C₇₋₁₃ aralkyloxy-carbonylamino group, a mono- or di- C₁₋₁₀ alkylsulfonylamino group (e.g., methylsulfonylamino, dimethylsulfonylamino), a C₆₋₁₄ arylsulfonylamino group and a C₁₋₆ alkoxy-carbamoylamino group (e.g., methoxycarbamoylamino)]; and
8) an amidino group;
   R¹ is an alkyl group having 4 to 10 carbon atoms (preferably isobutyl, neopentyl) or a cycloalkylalkyl group having 4 to 10 carbon atoms (preferably cyclopropylmethyl);
   R² is an aryl group having 6 to 14 carbon atoms (preferably phenyl), which optionally has 1 or 2 substituents selected from a halogen atom (preferably fluorine, chlorine) and C₁₋₆ alkyl (preferably methyl);
   X is a bond; and
   L is C₁₋₁₀ alkylene (preferably -CH₂-).

### (Compound I-b)

A compound wherein
ring A is a benzene ring optionally having 1 or 2 substituents selected from
1) a C₁₋₁₀ alkyl group (preferably ethyl) or a C₂₋₁₀ alkenyl group (preferably ethenyl) each optionally substituted by an alkoxycarbonyl group having 2 to 8 carbon atoms (preferably ethoxycarbonyl) or a carbamoyl group;
2) an optionally substituted hydroxy group [preferably, a C₁₋₁₀ alkoxy group (preferably methoxy) optionally substituted by a carbamoyl group; more preferably carbamoylmethoxy];
3) an acyl group (preferably carbamoyl, thiocarbamoyl, carboxyl);
4) an optionally substituted heterocyclic group [preferably, an aromatic heterocyclic group (preferably, furyl, thienyl, oxazolyl, oxadiazolyl, thiazolyl, tetrazolyl, pyridyl, pyrrolyl, triazolyl) optionally having 1 or 2 substituents selected from a C₁₋₆ alkyl group (preferably methyl), a carboxyl group, an alkoxycarbonyl group having 2 to 8 carbon atoms (preferably ethoxycarbonyl), a cyano group, a carbamoyl group, an amino group, a mono- or di-C₂₋₁₀ alkanoylamino group (e.g., acetylamino, isopentanoylamino), a C₁₋₁₀ alkoxy-carbonylamino group (e.g., methoxycarbonylamino), a carbamoylamino group, a mono- or di-C₁₋₁₀ alkyl-carbamoylamino group (e.g., methylcarbamoylamino, dimethylcarbamoylamino), a C₆₋₁₄ aryl-carbonylamino group (e.g., benzoylamino), a C₃₋₁₀ cycloalkyl-carbonylamino group, a C₇₋₁₃ aralkyloxy-carbonylamino group, a mono- or di-C₁₋₁₀ alkylsulfonylamino group (e.g., methylsulfonylamino, dimethylsulfonylamino), a C₆₋₁₄ arylsulfonylamino group and a C₁₋₆ alkoxy-carbamoylamino group (e.g., methoxycarbamoylamino), or a nonaromatic heterocyclic group (preferably, 5-oxooxadiazol-3-yl)];
   R¹ is an alkyl group having 4 to 10 carbon atoms (preferably isobutyl, neopentyl) or a cycloalkylalkyl group having 4 to 10 carbon atoms (preferably cyclopropylmethyl);
   R² is a C₁₋₁₀ alkyl group (preferably butyl) optionally substituted by 1 to 3 halogen atoms;
   X is -O-; and
   L is a C₁₋₁₀ alkylene (preferably -CH₂-).

Of the compounds represented by the formula (I), 2-[3-(aminomethyl)-4-butoxy-2-isobutyl-1-oxo-1,2-dihydro-6-isoquinolyl]-1,3-thiazole-4-carbonitrile;
2-[3-(aminomethyl)-4-butoxy-2-isobutyl-1-oxo-1,2-dihydro-6-isoquinolyl]-1,3-thiazole-4-carboxylic acid;
2-[3-(aminomethyl)-4-butoxy-2-isobutyl-1-oxo-1,2-dihydro-6-isoquinolyl]-1,3-thiazole-4-carboxamide; ethyl 2-[3-(aminomethyl)-4-butoxy-2-isobutyl-1-oxo-1,2-dihydro-6-isoquinolyl]-1,3-thiazole-4-carboxylate;
(E)-3-[3-(aminomethyl)-4-butoxy-2-isobutyl-1-oxo-1,2-dihydro-6-isoquinolyl]-2-propenamide;
(E)-3-[3-(aminomethyl)-2-isobutyl-4-phenyl-1-oxo-1,2-dihydro-6-isoquinolyl]-2-propenamide;
3-(aminomethyl)-2-isobutyl-1-oxo-4-phenyl-1,2-dihydro-6-isoquinolinecarboxamide;
2-{[3-(aminomethyl)-2-isobutyl-4-phenyl-1-oxo-1,2-dihydro-6-isoquinolyl]oxy}acetamide and the like are particularly preferable.

(2) A compound represented by the formula: 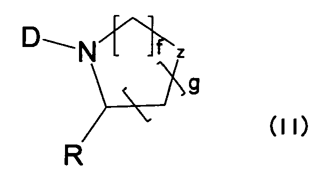 wherein
   f is 1 or 2; g is 0, 1 or 2; Z is -CH₂-, -O-, -S-, -SO-, -SO₂-or -NR⁴- (R⁴ is a hydrogen atom or a C₁₋₆ alkyl group); R is a hydrogen atom, a cyano group, -CHO, -B(OH)₂, -P(O)(OR⁴), -CCR⁵ or -CH=NR⁶ (R⁵ is a hydrogen atom, a fluorine atom, a C₁₋₆ alkyl group, a cyano group, a nitro group, -OR⁴, -CO₂R⁴ or -COR⁴ (R⁴ is as defined above); R⁶ is a phenyl group, a hydroxy group, - OR⁴, -OCOR⁴ or a benzyloxy group (R⁴ is as defined above)); and D is an optionally substituted amino acid residue, which is described in WO95/15309 and the like, or a salt thereof.

In the formula, the C₁₋₆ alkyl group for R⁴ is, for example, methyl, ethyl, propyl, isopropyl, butyl, isobutyl, sec.-butyl, t.-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl and the like.

The amino acid residue of the "optionally substituted amino acid residue" for D includes, for example, a group obtained by eliminating OH of the carboxyl group constituting α-amino acid or β-amino acid from the amino acid.

Here, as the α-amino acid, for example, alanine, arginine, asparagine, aspartic acid, glutamine, glutamic acid, glycine, histidine, isoleucine, leucine, lysine, methionine, phenylalanine, proline, serine, threonine, tryptophan, tyrosine, valine, citrulline, ornithine, homocysteine and the like can be mentioned.

As the β-amino acid, for example, β-alanine, β-aminocyclopropanoic acid, β-aminocyclobutanoic acid, β-aminocyclopentanoic acid, β-aminocyclohexanoic acid, β-aminocycloheptanoic acid and β-aminocyclooctanoic acid can be mentioned. The β-amino acid may have unsaturated bond(s) in the carbon chain constituting the amino acid.

While the above-mentioned α-amino acid and β-amino acid may be any of a D form, an L form and a DL form, a naturally occurring L form is preferable.

The above-mentioned amino acid residue may have 1 or 2 substituents on an amino group or an amino acid side chain constituting the amino acid.

As the above-mentioned "substituent on an amino group", an optionally substituted hydrocarbon group, an optionally substituted piperidinyl group and the like are preferable.

As the hydrocarbon group of the "optionally substituted hydrocarbon group", for example, a C₁₋₆ alkyl group, a C₃₋₁₂ cycloalkyl group, a C₂₋₆ alkenyl group, a C₃₋₁₂ cycloalkenyl group, a C₂₋₆ alkynyl group, a C₄₋₁₂ cycloalkadienyl group, a C₆₋₁₄ aryl group (preferably a phenyl group), a C₇₋₁₅ aralkyl group (preferably a benzyl group, a phenethyl group), an adamantyl group, a bicyclo[2.2.1]heptyl group, a bicyclo[3.1.1]heptyl group and the like can be mentioned.

The hydrocarbon group may have 1 to 3 substituents at substitutable position(s), and as such substituent, for example, a halogen atom (preferably fluorine, chlorine); a cyano group; a hydroxy group optionally substituted by an acyl group; a hydroxymethyl group; a C₁₋₆ alkoxy group optionally substituted by 1 to 3 halogen atoms (preferably fluorine); and an amino group optionally mono- or di-substituted by an optionally substituted C₆₋₁₄ aryl group or an optionally substituted heterocyclic group, can be mentioned.

As the acyl group of the "hydroxy group optionally substituted by acyl group", for example, an acyl group recited as an example of the substituent of ring A in the aforementioned compound (I-a) can be mentioned.

As the C₆₋₁₄ aryl group of the "optionally substituted C₆₋₁₄ aryl group", for example, a phenyl group, a naphthyl group and the like can be mentioned.

As the heterocyclic group of the "optionally substituted heterocyclic group", for example, a pyridyl group, a pyrimidyl group, a pirazyl group, a quinolyl group, an isoquinolyl group, a quinoxalyl group and the like can be mentioned.

The C₆₋₁₄ aryl group and the heterocyclic group may have 1 to 3 substituents at substitutable position(s), and as such substituent, for example, a halogen atom (preferably fluorine, chlorine, bromine); a cyano group; a nitro group; a C₁₋₆ alkyl group; a C₁₋₆ alkoxy group optionally substituted by 1 to 3 halogen atoms (preferably fluorine); a carboxyl group; a carbamoyl group; a C₁₋₆ alkylsulfonyl group (preferably a methanesulfonyl group); an aminosulfonyl group (preferably a dimethylaminosulfonyl group) optionally mono- or di-substituted by C₁₋₆ alkyl group(s) and the like can be mentioned.

The substituent of the aforementioned "optionally substituted hydrocarbon group" is particularly preferably a 5-nitro-2-pyridylamino group, a 5-cyano-2-pyridylamino group, a 2-pyrimidylamino group, a 2-pirazylamino group and the like.

As the substituent of the aforementioned "optionally substituted piperidinyl group", for example, a C₁₋₆ alkyl group; a hydroxymethyl group; an "optionally substituted C₆₋₁₄ aryl group" and an "optionally substituted heterocyclic group" recited as examples of the aforementioned "amino group optionally mono- or di-substituted by an optionally substituted C₆₋₁₄ aryl group or an optionally substituted heterocyclic group" can be mentioned. The number of the substituents is, for example, 1 to 3.

As the above-mentioned "substituent on the amino acid side chain", for example, an optionally substituted hydrocarbon group, a hydroxy group, a C₁₋₁₀ alkoxy group optionally substituted by 1 to 3 halogen atoms (preferably fluorine), an acyl group, an optionally substituted amino group and the like can be mentioned.

Here, as the hydrocarbon of the "optionally substituted hydrocarbon group", for example, a C₁₋₁₀ alkyl group, a C₃₋₁₂ cycloalkyl group, a C₂₋₁₀ alkenyl group, a C₃₋₁₂ cycloalkenyl group and the like can be mentioned.

The hydrocarbon group may have 1 to 3 substituents at substitutable position(s) and, as such substituent, for example, an amino group, a C₁₋₆ alkyl-carbonylamino group (preferably an acetylamino group), a hydroxy group, a C₁₋₆ alkoxy group, a heterocyclic group (preferably pyridyl) and the like can be mentioned.

As the above-mentioned "acyl group", an optionally substituted nitrogen-containing heterocycle-carbonyl group is preferable. As the "optionally substituted nitrogen-containing heterocycle", for example, a nitrogen-containing heterocycle (preferably pyridine, pyridazine, pyrimidine, pyrazine, imidazole, pyrazole, thiazole, isothiazole, oxazole, isoxazole) optionally having 1 to 3 substituents selected from a halogen atom (preferably fluorine, chlorine, bromine), a cyano group, a nitro group, a C₁₋₆ alkyl group (e.g., a trifluoromethyl group) optionally substituted by 1 to 3 halogen atoms (preferably fluorine), a C₁₋₆ alkoxy group, an amino group optionally mono- or di-substituted by C₁₋₆ alkyl group(s), a hydroxy group, a carboxyl group and a C₁₋₆ alkyl-oxycarbonyl group, and the like can be mentioned.

As the substituent of the above-mentioned "optionally substituted amino group", for example, a C₁₋₆ alkyl group optionally having 1 to 3 substituents selected from a carboxyl group, a carbamoyl group, a C₁₋₆ alkyl-oxycarbonyl group and a nitrogen-containing heterocyclic group (preferably pyridyl) and the like can be mentioned. These substituents may be bonded to a hydroxy group, a carboxyl group, an amino group and the like on the amino acid side chain.

As a salt of the compound represented by the formula (II), those similar to the salts of the compound represented by the formula (I) can be mentioned.

The compound represented by the formula (II) may be an anhydrate or a hydrate, or a prodrug.

Preferable examples of the compound represented by the formula (II) include N-(N'-substituted glycyl)-2-cyanopyrrolidine derivatives such as (2S)-1-{{{2-[(5-cyanopyridin-2-yl)amino]ethyl}amino}acetyl}-2-cyano-pyrrolidine (DPP-728) (described in WO98/19998) represented by the formula 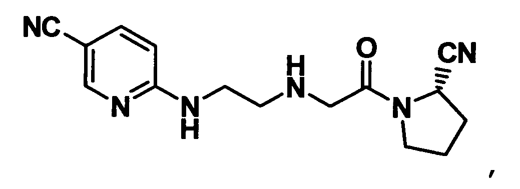 (2S)-1-{[(3-hydroxy-1-adamantyl)amino]acetyl}-2-cyanopyrrolidine (LAF237, Vildagliptin) (described in WO00/34241) represented by the formula 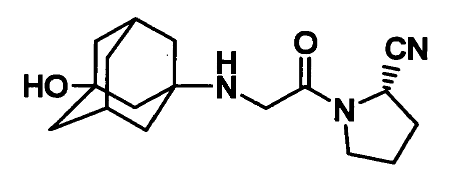 and the like;
thiazolidine or pyrrolidine derivatives (described in WO01/72290 and the like) such as L-threo-isoleucyl thiazolidine represented by the formula 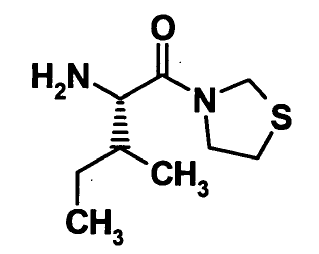 and 1/2 fumarate thereof (P32/98), L-allo-isoleucyl thiazolidine, L-threo-isoleucyl pyrrolidine, L-allo-isoleucyl pyrrolidine, L-valyl pyrrolidine and the like;
a compound represented by the formula 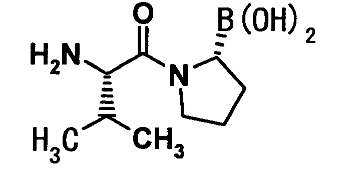 (PT-100); P93/01 and the like can be mentioned.
(3) A compound represented by the formula: 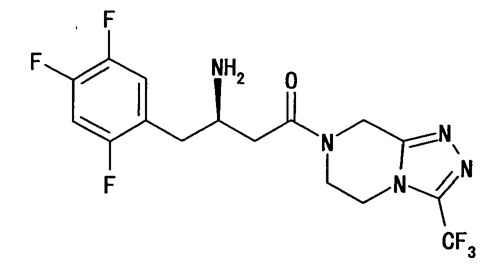 and a salt thereof (preferably phosphate, hydrochloride) (MK-431, Sitagliptin phosphate).
(4) A compound represented by the formula: 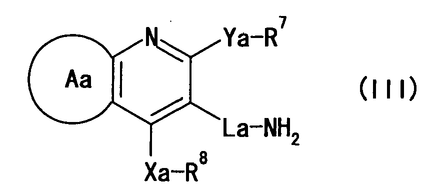 wherein
   ring Aa is an optionally substituted 5- to 10-membered aromatic ring,
   R⁷ and R⁸ are the same or different and each is an optionally substituted hydrocarbon group or an optionally substituted heterocyclic group,
   Xa and Ya are the same or different and each is a bond, -0-, - S-, -SO-, -SO₂- or -NR⁹- (R⁹ is a hydrogen atom or an optionally substituted hydrocarbon group); and
   La is a divalent hydrocarbon group, which is described in WO2004/014860, or a salt thereof.

As a salt of the compound represented by the formula (III), those similar to the salts of the compound represented by the formula (I) can be mentioned.

The compound represented by the formula (III) may be an anhydrate or a hydrate, or a prodrug.

Preferable examples of a compound represented by the formula (III) include the following compounds.

### (Compound III-a)

A compound wherein ring Aa is a benzene ring optionally having 1 or 2 substituents selected from;
1) a halogen atom (e.g., fluorine, chlorine, bromine, iodine);
2) a nitro group;
3) a cyano group;
4) an alkylenedioxy group having 1 to 3 carbon atoms (e.g., methylenedioxy);
5) an alkyl group having 1 to 10 carbon atoms (e.g., methyl, ethyl, propyl, butyl, pentyl) or an alkenyl group having 2 to 10 carbon atoms (e.g., ethenyl, 3-butenyl), each of which optionally has 1 to 3 substituents selected from a halogen atom, a hydroxy group, a carboxyl group, an alkoxycarbonyl group having 2 to 8 carbon atoms (e.g., ethoxycarbonyl), a carbamoyl group, a cyano group, an amino group, an alkylcarbonylamino group having 2 to 8 carbon atoms (e.g., acetylamino, isobutanoylamino), an alkoxycarbonylamino group having 2 to 8 carbon atoms (e.g., methoxycarbonylamino, ethoxycarbonylamino), an alkylsulfonylamino group having 1 to 8 carbon atoms (e.g., methylsulfonylamino), an alkylcarbamoylamino group having 2 to 8 carbon atoms (e.g., methylcarbamoylamino), a carboxyl-C₁₋₆ alkylthio group (e.g., carboxylmethylthio), an (alkoxycarbonyl having 2 to 8 carbon atoms)-C₁₋₆ alkylthio group (e.g., ethoxycarbonylmethylthio) and a carbamoyl-C₁₋₆ alkylthio group (e.g., carbamoylmethylthio);
6) an optionally substituted hydroxy group [e.g., an alkoxy group having 1 to 10 carbon atoms (e.g., methoxy, ethoxy, propoxy, isopropoxy, butoxy, isobutoxy, pentyloxy), a cycloalkyloxy group having 3 to 10 carbon atoms (e.g., cyclopentyloxy) or an aralkyloxy group having 7 to 13 carbon atoms (e.g., benzyloxy),
   each optionally having 1 to 3 substituents selected from a halogen atom; an alkoxy group having 1 to 3 carbon atoms optionally substituted by 1 or 2 substituents selected from a carboxyl group and an alkoxycarbonyl group having 2 to 5
   carbon atoms (e.g., tert-butoxycarbonyl) (e.g., methoxy, carboxylmethoxy, tert-butoxycarbonylmethoxy); an alkoxycarbonyl group having 2 to 5 carbon atoms (e.g., methoxycarbonyl, ethoxycarbonyl); an alkylcarbonyl group having 2 to 5 carbon atoms (e.g., pivaloyl); a cyano group; a carbamoyl group optionally substituted by 1 or 2 substituents selected from a C₁₋₁₀ alkyl group (e.g., methyl, ethyl, propyl, isopropyl) and a C₁₋₁₀ alkylsulfonyl group (e.g., methylsulfonyl); a hydroxy group; a carboxyl group; an amino group; an alkylcarbonylamino group having 2 to 5 carbon atoms (e.g., acetylamino); an aromatic heterocyclic group (e.g., furyl, thienyl, oxazolyl, thiazolyl, isoxazolyl, tetrazolyl, oxadiazolyl, thiadiazolyl) optionally substituted by 1 to 3 substituents selected from a C₁₋₆ alkyl group (e.g., methyl, ethyl) and a C₂₋₈ alkoxycarbonyl group (e.g., methoxycarbonyl, ethoxycarbonyl); and a cycloalkyl group having 3 to 10 carbon atoms (e.g., cyclopropyl, cyclohexyl);
   a hydroxy group];
7) an acyl group [e.g., formyl, carboxyl, C₁₋₆ alkyl-carbonyl (e.g., acetyl), C₁₋₆ alkoxy-carbonyl (e.g., methoxycarbonyl), carbamoyl, aminocarbamoyl, hydroxycarbamoyl, mono- or di- (C₁₋₆ alkyl optionally having 1 to 3 substituents selected from halogen atom and C₁₋₆ alkoxy-carbonyl (e.g., ethoxycarbonyl))-carbamoyl (e.g., methylcarbamoyl, ethylcarbamoyl, propylcarbamoyl, dimethylcarbamoyl, trifluoroethylcarbamoyl, ethoxycarbonylmethylcarbamoyl), C₃₋₁₀ cycloalkyl-carbamoyl (e.g., cyclopropylcarbamoyl), C₇₋₁₃ aralkyl-carbamoyl (e.g., benzylcarbamoyl), nitrogen-containing heterocycle-carbonyl (e.g., pyrrolidinylcarbonyl, piperidinocarbonyl) optionally substituted by hydroxy, C₁₋₆ alkylsulfonyl (e.g., methylsulfonyl), C₁₋₆ alkylsulfinyl (e.g., methylsulfinyl), thiocarbamoyl];
8) an optionally substituted amino group [e.g., amino, mono-or di-C₂₋₁₀ alkylcarbonylamino (e.g., acetylamino, propionylamino, isobutanoylamino, isopentanoylamino), C₁₋₁₀ alkoxy-carbonylamino (e.g., methoxycarbonylamino), carbamoylamino, mono- or di-C₁₋₁₀ alkyl-carbamoylamino (e.g., methylcarbamoylamino, dimethylcarbamoylamino), C₆₋₁₄ aryl-carbonylamino (e.g., benzoylamino), C₃₋₁₀ cycloalkyl-carbonylamino (e.g., cyclopentylcarbonylamino), C₇₋₁₃ aralkyloxy-carbonylamino (e.g., benzyloxycarbonylamino), mono-or di-C₁₋₁₀ alkylsulfonylamino (e.g., methylsulfonylamino, dimethylsulfonylamino), C₆₋₁₄ arylsulfonylamino (e.g., phenylsulfonylamino), C₁₋₆ alkoxy-carbamoylamino (e.g., methoxycarbamoylamino), carbamoyl-C₁₋₁₀ alkylamino (e.g., carbamoylmethylamino), C₂₋₅ alkoxycarbonyl-C₁₋₁₀ alkylamino (e.g., methoxycarbonylmethylamino, tert-butoxycarbonylmethylamino)];
9) an optionally substituted cycloalkyl group having 3 to 10 carbon atoms [e.g., a cycloalkyl group having 3 to 10 carbon atoms (e.g., cyclopropyl, cyclobutyl), which optionally has 1 to 3 substituents selected from a C₁₋₆ alkyl group optionally substituted by 1 to 3 halogen atoms (e.g., methyl, trifluoromethyl), a carboxyl group, an alkoxycarbonyl group having 2 to 8 carbon atoms (e.g., methoxycarbonyl, ethoxycarbonyl, isopropoxycarbonyl), a cyano group, a carbamoyl group, an amino group, a mono- or di-C₂₋₁₀ alkylcarbonylamino group (e.g., acetylamino, isopentanoylamino), a C₁₋₁₀ alkoxy-carbonylamino group (e.g., methoxycarbonylamino), a carbamoylamino group, a mono- or di-C₁₋₁₀ alkyl-carbamoylamino group (e.g., methylcarbamoylamino, dimethylcarbamoylamino), a C₆₋₁₄ aryl-carbonylamino group (e.g., benzoylamino), a C₃₋₁₀ cycloalkyl-carbonylamino group, a C₇₋₁₃ aralkyloxy-carbonylamino group, a mono- or di-C₁₋₁₀ alkylsulfonylamino group (e.g., methylsulfonylamino, dimethylsulfonylamino), a C₆₋₁₄ arylsulfonylamino group and a C₁₋₆ alkoxy-carbamoylamino group (e.g., methoxycarbamoylamino)];
10) an aryl group having 6 to 14 carbon atoms (e.g., phenyl);
11) an optionally substituted thiol group [e.g., an alkylthio group having 1 to 10 carbon atoms (e.g., methylthio), which is optionally substituted by a carbamoyl group];
12) an optionally substituted heterocyclic group [e.g., an aromatic heterocyclic group (preferably, furyl, thienyl, oxazolyl, oxadiazolyl, thiazolyl, tetrazolyl, pyridyl, pyrrolyl, triazolyl) or a nonaromatic heterocyclic group (preferably, dioxoisoindol-2-yl; 5-oxooxadiazol-3-yl; 5-oxothiadiazol-3-yl; 3-oxopiperazin-1-yl; 2,3-dioxopiperazin-1-yl; 2,5-dioxopiperazin-1-yl), each optionally having 1 or 2 substituents selected from a C₁₋₆ alkyl group optionally substituted by 1 to 3 halogen atoms (e.g., methyl, trifluoromethyl), a carboxyl group, an alkoxycarbonyl group having 2 to 8 carbon atoms (e.g., methoxycarbonyl, ethoxycarbonyl, isopropoxycarbonyl, tert-butoxycarbonyl), a cyano group, a carbamoyl group, an amino group, a mono- or di-C₂₋₁₀ alkylcarbonylamino group (e.g., acetylamino, isopentanoylamino), a C₁₋₁₀ alkoxy-carbonylamino group (e.g., methoxycarbonylamino), a carbamoylamino group, a mono- or di-C₁₋₁₀ alkyl-carbamoylamino group (e.g., methylcarbamoylamino, dimethylcarbamoylamino), a C₆₋₁₄ aryl-carbonylamino group (e.g., benzoylamino), a C₃₋₁₀ cycloalkyl-carbonylamino group, a C₇₋₁₃ aralkyloxy-carbonylamino group, a mono- or di- C₁₋₁₀ alkylsulfonylamino group (e.g., methylsulfonylamino, dimethylsulfonylamino), a C₆₋₁₄ arylsulfonylamino group, a C₁₋₆ alkoxy-carbamoylamino group (e.g., methoxycarbamoylamino), an alkylcarbonyl group having 2 to 5 carbon atoms (e.g., acetyl) and a carbamoyl-C₁₋₆ alkyl group (e.g., carbamoylmethyl)]; or
13) an amidino group;
   R⁷ is an alkyl group having 3 to 10 carbon atoms (preferably isobutyl) or a cycloalkylalkyl group having 4 to 10 carbon atoms (preferably cyclopropylmethyl);
   R⁸ is an alkyl group having 1 to 10 carbon atoms (e.g., methyl, ethyl, propyl, isopropyl, butyl, isobutyl, sec-butyl, tert-butyl, pentyl), an aryl group having 6 to 14 carbon atoms (e.g., phenyl) or an aralkyl group having 7 to 13 carbon atoms (e.g., benzyl, phenethyl, naphthylmethyl), each of which may have 1 to 3 (preferably 1 or 2) substituents selected from a halogen atom (e.g., fluorine, chlorine), a hydroxy group, a nitro group, an amino group, an optionally halogenated alkyl group having 1 to 6 carbon atoms (e.g., trifluoromethyl, methyl), an alkoxy group having 1 to 6 carbon atoms (e.g., methoxy), an aromatic heterocyclic group (e.g., quinolyl, thienyl) and a cycloalkyl group having 3 to 10 carbon atoms (e.g., cyclopentyl);
   Xa is a bond;
   Ya is a bond; and
   La is C₁₋₁₀ alkylene.

Of the compounds represented by the formula (III), (2E)-3-[3-(aminomethyl)-2-isobutyl-4-(4-methylphenyl)quinolin-6-yl]acrylamide;
5-[[3-(aminomethyl)-2-isobutyl-4-(4-methylphenyl)quinolin-6-yl]oxy]pentanoic acid;
4-[3-(aminomethyl)-2-isobutyl-4-(4-methylphenyl)quinolin-6-yl]piperazin-2-one;
1-[3-(aminomethyl)-2-isobutyl-4-(4-methylphenyl)quinolin-6-yl]piperazine-2,5-dione and the like are particularly preferable.

(5) A compound represented by the formula: 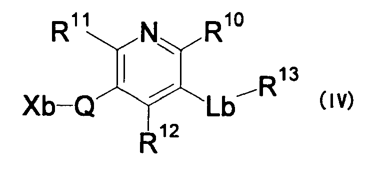
wherein
R¹⁰ and R¹¹ are the same or different and each is an optionally substituted hydrocarbon group or an optionally substituted hydroxy group,
R¹² is an optionally substituted aromatic group,
R¹³ is an optionally substituted amino group,
Lb is a divalent chain hydrocarbon group,
Q is a bond or a divalent chain hydrocarbon group, and
Xb is a hydrogen atom, a cyano group, a nitro group, an acyl group, a substituted hydroxy group, an optionally substituted thiol group, an optionally substituted amino group or an optionally substituted cyclic group,
provided that when Xb is an ethoxycarbonyl group, then Q is a divalent chain hydrocarbon group (except
2,6-diisopropyl-3-methylaminomethyl-4-(4-fluorophenyl)-5-pentylpyridine;
2,6-diisopropyl-3-aminomethyl-4-(4-fluorophenyl)-5-pentylpyridine;
2,6-diisopropyl-3-(dimethylamino)methyl-4-(4-fluorophenyl)-5-pentylpyridine;
2,6-diisopropyl-3-(ethylamino)methyl-4-(4-fluorophenyl)-5-pentylpyridine; and
3-(tert-butyldimethylsilyloxymethyl)-2,6-diisopropyl-4-(4-fluorophenyl)-5-indolyl-5-aminomethyl)pyridine), which is described in WO2005/042488, or a salt thereof.

As a salt of the compound represented by the formula (IV), those similar to the salts of a compound represented by the formula (I) can be mentioned.

The compound represented by the formula (IV) may be an anhydrate or a hydrate, or a prodrug.

Preferable examples of a compound represented by the formula (IV) include the following compounds.

### (Compound IV')

A compound wherein
R¹⁰ and R¹¹ are the same or different and each is
(1) a C₁₋₁₀ alkyl group optionally substituted by 1 to 3 substituents selected from a C₃₋₁₀ cycloalkyl group (preferably cyclopropyl), a C₁₋₆ alkoxy-carbonyl group, a C₁₋₆ alkoxy group and the like;
(2) a C₆₋₁₄ aryl group (preferably phenyl) optionally substituted by 1 to 3 substituents selected from a halogen atom, a carboxyl group, a C₁₋₆ alkoxy-carbonyl group, a carbamoyl group and the like; or
(3) a C₇₋₁₃ aralkyl group (preferably benzyl);
   R¹² is a C₆₋₁₄ aryl group (preferably phenyl) optionally substituted by 1 to 3 substituents selected from a C₁₋₆ alkyl group optionally substituted by 1 to 3 halogen atoms, a halogen atom, a C₁₋₆ alkoxy-carbonyl group, a carboxyl group, a hydroxy group, a C₁₋₆ alkoxy group optionally substituted by 1 to 3 halogen atoms, and the like;
   R¹³ is an amino group optionally mono- or di- substituted by C₁₋₆ alkyl group(s) (preferably an amino group);
   Lb is a C₁₋₁₀ alkylene (preferably -CH₂-);
   Q is a bond, a C₁₋₁₀ alkylene or a C₂₋₁₀ alkenylene (preferably a bond, -CH₂-, -(CH₂)₂-, -CH=CH-); and
   Xb is
      (1) a hydrogen atom;
      (2) a cyano group;
      (3) (3a) a carboxyl group;
         (3b) a carbamoyl group;
         (3c) a C₁₋₆ alkoxy-carbonyl group optionally substituted by substituent(s) selected from a carboxyl group, a carbamoyl group, a thiocarbamoyl group, a C₁₋₆ alkoxy-carbonyl group and a C₁₋₆ alkyl-carbonyloxy group;
         (3d) an aromatic heterocycle (preferably pyridyl, thiazolyl, oxazolyl, indolyl)-C₁₋₆ alkoxy-carbonyl group optionally substituted by substituent(s) selected from a carboxyl group, a carbamoyl group, a thiocarbamoyl group and a C₁₋₆ alkoxy-carbonyl group;
         (3e) a non-aromatic heterocycle (preferably oxodioxolyl, oxodioxolanyl, oxo-2-benzofuranyl)-C₁₋₆ alkoxy-carbonyl group optionally substituted by a C₁₋₆ alkyl group;
         (3f) a C₇₋₁₃ aralkyloxy-carbonyl group optionally substituted by substituent(s) selected from a carboxyl group, a carbamoyl group, a thiocarbamoyl group and a C₁₋₆ alkoxy-carbonyl group;
         (3g) a carbamoyl group mono- or di-substituted by a C₁₋₆ alkyl group optionally substituted by substituent(s) selected from 1 to 3 halogen atoms and a C₁₋₆ alkoxy group;
         (3h) a carbamoyl-C₁₋₆ alkyl-carbamoyl group optionally mono-or di-substituted by a C₁₋₆ alkyl group optionally substituted by 1 to 3 halogen atoms;
         (3i) a C₁₋₆ alkoxy-carbonyl-C₁₋₆ alkyl-carbamoyl group optionally substituted by a C₁₋₆ alkyl group;
         (3j) a mono- or di-C₃₋₁₀ cycloalkyl-carbamoyl group optionally substituted by a C₁₋₆ alkyl group;
         (3k) a C₇₋₁₃ aralkyl-carbamoyl group optionally substituted by substituent(s) selected from a halogen atom, a hydroxy group, a C₁₋₆ alkoxy-carbonyl group and a C₁₋₆ alkyl group;
         (31) an aromatic heterocyclic (preferably pyridyl, thiazolyl, oxazolyl, indolyl)-C₁₋₆ alkyl-carbamoyl group;
         (3m) a C₁₋₆ alkylsulfonyl group optionally substituted by substituent(s) selected from a carboxyl group, a carbamoyl group and a C₁₋₆ alkoxy-carbonyl group;
         (3n) a C₆₋₁₄ arylsulfonyl group optionally substituted by substituent(s) selected from a C₁₋₆ alkyl group, a carboxyl group, a carbamoyl group, a thiocarbamoyl group, a C₁₋₆ alkoxy-carbonyl group and a C₁₋₆ alkylsulfonyl group;
         (3o) a nitrogen-containing heterocyclic (preferably pyrrolidinyl, piperidino, piperazinyl, morpholino)-carbonyl group optionally substituted by substituent(s) selected from a hydroxy group and a C₁₋₆ alkoxy-carbonyl group;
         (3p) a C₆₋₁₄ aryl-nitrogen-containing heterocycle (preferably pyrrolidinyl, piperidino, piperazinyl, morpholino)-carbonyl group optionally substituted by halogen atom(s);
         (3q) a C₇₋₁₃ aralkyl-nitrogen-containing heterocycle (preferably pyrrolidinyl, piperidino, piperazinyl, morpholino)-carbonyl group optionally substituted by halogen atom(s);
         (3r) a non-aromatic heterocycle (preferably oxodioxolyl, oxodioxolanyl, oxo-2-benzofuranyl)oxy-carbonyl group; or
         (3s) a phosphono group optionally mono- or di-substituted by a C₁₋₆ alkyl group;
      (4) a C₁₋₆ alkyl-carbonyloxy group;
      (5) (5a) a C₁₋₆ alkylthio group optionally substituted by substituent(s) selected from a carboxyl group, a carbamoyl group and a C₁₋₆ alkoxy-carbonyl group;
         (5b) a C₆₋₁₄ arylthio group (preferably phenylthio) optionally substituted by substituent(s) selected from a carboxyl group, a C₁₋₆ alkoxy-carbonyl group and a C₁₋₆ alkylthio group; or
         (5c) a 5-membered aromatic heterocyclylthio group (preferably thiazolylthio, oxazolylthio, triazolylthio) optionally substituted by a C₁₋₆ alkyl group;
      (6) (6a) an amino group;
         (6b) a C₁₋₆ alkoxy-carbonyl-C₁₋₁₀ alkylamino group (preferably methoxycarbonylmethylamino, ethoxycarbonylmethylamino, tert-butoxycarbonylmethylamino);
         (6c) a carboxy-C₁₋₁₀ alkylamino group;
         (6d) a C₇₋₁₃ aralkyloxy-carbonylamino group;
         (6e) a carbamoylamino group;
         (6f) a mono- or di-C₁₋₆ alkyl-carbamoylamino group;
         (6g) a C₁₋₆ alkylsulfonylamino group;
         (6h) a C₆₋₁₄ arylsulfonylamino group optionally substituted by a C₁₋₆ alkylsulfonyl group; or
         (6i) an aromatic heterocycle (e.g., pyridyl, thiazolyl, oxazolyl, indolyl)-sulfonylamino group optionally substituted by substituent(s) selected from a C₁₋₆ alkyl group and a mono- or di-(C₁₋₆ alkyl-carbonyl)-amino group; or
      (7) tetrazolyl, oxoimidazolidinyl (preferably 2-oxoimidazolidin-1-yl), dioxoimidazolidinyl (preferably 2,4-dioxoimidazolidin-3-yl), oxopiperazinyl (preferably 3-oxopiperazin-1-yl), dioxopiperazinyl (preferably 2,3-dioxopiperazin-1-yl, 2,5-dioxopiperazin-1-yl) or oxodihydrooxadiazolyl (preferably 5-oxo-4,5-dihydro-1,2,4-oxadiazol-3-yl).

A compound represented by the formula (IV) can be produced by a method known per se, for example, the methods described in detail in the following, or a method analogous thereto.

In the formula (IV), compound (IV-1)
wherein
Lb is Lba(CH₂) (Lba- is a bond or a divalent chain hydrocarbon group),
Xb is Xba (Xba is a hydrogen atom, a nitro group, an acyl group, a substituted hydroxy group, an optionally substituted thiol group, an optionally substituted amino group or an optionally substituted cyclic group), and
R¹³ is an amino group,
can be produced by the following method A or a method analogous thereto.

Here, as the divalent chain hydrocarbon group for Lba, those similar to the aforementioned Lb can be mentioned. Lba is preferably a bond or a C₁₋₉ alkylene.

As the acyl group, substituted hydroxy group, optionally substituted thiol group, optionally substituted amino group and optionally substituted cyclic group for Xba, those similar to the aforementioned Xb can be respectively used. 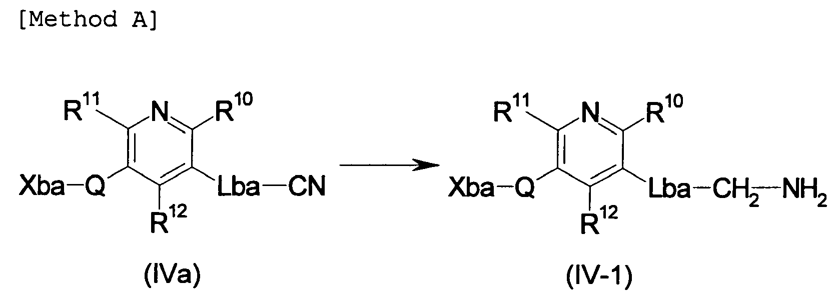 wherein the symbols in the formulas are as defined above, provided when Xba is an ethoxycarbonyl group, then Q is a divalent chain hydrocarbon group.

According to this method, compound (IVa) is subjected to a reduction reaction to give compound (IV-1).

The reduction reaction is carried out in a solvent that does not adversely influence the reaction, in the presence of a reducing agent according to a conventional method.

As the reducing agent, for example, metal hydrogen compounds such as bis(2-methoxyethoxy)aluminumsodium hydride, diisobutylaluminum hydride and the like; metal hydrogen complex compounds such as sodium borohydride, sodium cyanoborohydride, aluminum lithium hydride, aluminum sodium hydride and the like; and the like can be mentioned.

The amount of the reducing agent to be used is generally 0.1 to 20 molar equivalents relative to compound (IVa).

As a solvent that does not adversely influence the reaction, for example, alcohols such as methanol, ethanol, propanol, 2-propanol, butanol, isobutanol, tert-butanol and the like; aromatic hydrocarbons such as benzene, toluene, xylene and the like; aliphatic hydrocarbons such as hexane, heptane and the like; ethers such as diethyl ether, diisopropyl ether, tert-butyl methyl ether, tetrahydrofuran, dioxane, dimethoxyethane and the like; esters such as methyl acetate, ethyl acetate, n-butyl acetate, tert-butyl acetate and the like; amides such as dimethylformamide, dimethylacetamide, N-methylpyrrolidone and the like are used. Two or more kinds of the solvents may be used in a mixture at an appropriate ratio.

The reaction temperature is generally -70°C to 150°C, preferably -20°C to 100°C.

The reaction time is generally 0.1 hr to 100 hr, preferably 0.1 hr to 40 hr.

The reduction reaction can also be carried out in the presence of a metal catalyst such as palladium-carbon, palladium black, palladium chloride, platinum oxide, platinum black, platinum-palladium, Raney-nickel, Raney cobalt and the like, and a hydrogen source, in a solvent that does not adversely influence the reaction.

The amount of the metal catalyst to be used is generally 0.001 molar equivalent to 1000 molar equivalents, preferably 0.01 molar equivalent to 100 molar equivalents, relative to compound (IVa).

As a hydrogen source, for example, hydrogen gas, formic acid, formic acid amine salt, phosphine acid salt, hydrazine and the like can be mentioned.

As a solvent that does not adversely influence the reaction, those recited for the aforementioned reduction reaction using a reducing agent can be mentioned.

The reaction temperature and the reaction time are similar to those in the aforementioned reduction reaction using a reducing agent.

This reaction may be carried out, where necessary, in the presence of ammonia (e.g., aqueous ammonia, ammonia-ethanol). By the reaction in the presence of ammonia, side reaction is suppressed and compound (IV-1) can be produced in a high yield.

The thus-obtained compound (IV-1) can be isolated and purified by a known separation and purification means, for example, concentration, concentration under reduced pressure, solvent extraction, crystallization, recrystallization, phase transfer, chromatography and the like.

Compound (IVa) to be used as a starting material compound in the above-mentioned method A can be produced according to a method known per se.

For example, compound (IVa-1) of the formula (IVa), wherein Q and Lba are bonds and Xba is an acyl group, can be produced by the following method B. 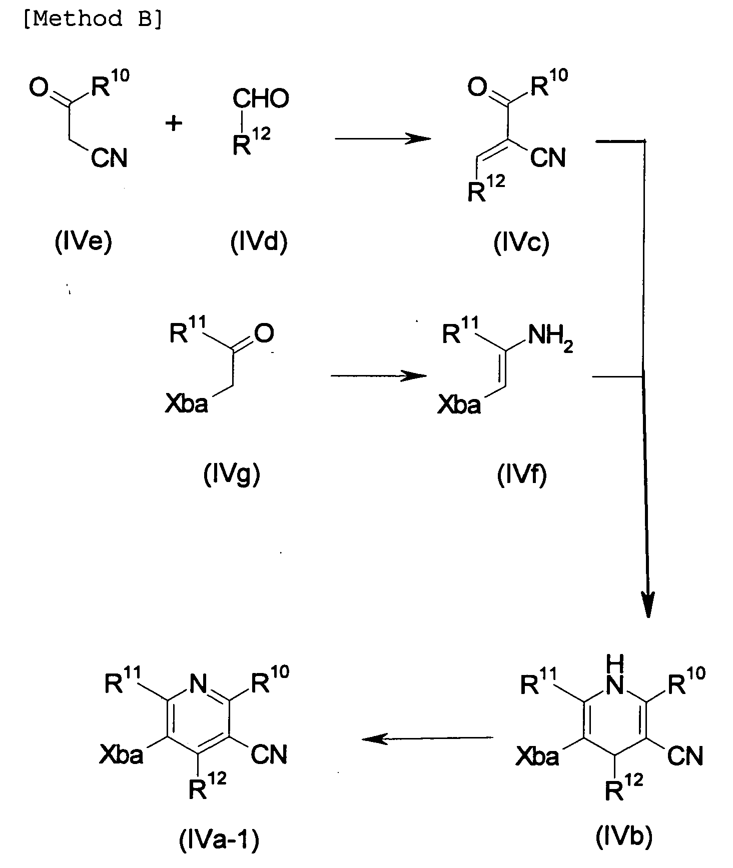 wherein the symbols in the formula are as defined above.

Compound (IVa-1) can be produced by a method known per se, for example, a reaction of compound (IVb) and an oxidant such as diluted nitric acid, diammonium cerium nitrate and the like in a solvent that does not adversely influence the reaction such as 1,4-dioxane, acetone and the like.

The compound (IVb) can be produced, for example, from compound (IVc) and compound (IVf) by a method known *per se*, for example, pyridine synthetic method of Hantzch described in "Courses in Experimental Chemistry, Ed (the Chemical Society of Japan)" Maruzen Press (1973), vol. 14, Synthesis and Reaction of Organic Compound, page 2057, or a method analogous thereto.

Compound (IVc) can be produced by a method known *per se*, for example, by applying compound (IVd) and compound (IVe) to a known Knoevenagel method.

Compound (IVf) can be produced from compound (IVg) according to a method known per se, for example, the method described in Synthesis, (1999), vol. 11, pages 1951-1960; Journal of Chemical Society Perkin Transactions 1, (2002), pages 1663-1671 and the like, or a method analogous thereto.

The aforementioned compound (IVd), compound (IVe) and compound (IVg) can be produced according to a method known per se.

Compound (IV-2) of the formula (IV) wherein R¹³ is an amino group mono- or di- substituted by C₁₋₁₀ alkyl group(s) can be produced by subjecting compound (IV-3) of the formula (IV) wherein R¹³ is an amino group to an alkylation reaction.

This reaction is carried out according to a conventional method
(1) using an alkylating agent in a solvent that does not adversely influence the reaction and, where necessary, in the presence of a base or
(2) using a carbonyl compound in a solvent that does not adversely influence the reaction and, where necessary, in the presence of a reducing agent.

Here, as the alkylating agent, for example, C₁₋₁₀ alkyl halides, C₁₋₁₀ alkylsulfonic acid esters and the like can be mentioned.

As the carbonyl compound, for example, aldehydes, ketones and the like can be mentioned.

The amount of the alkylating agent and carbonyl compound to be used is preferably about 1 molar equivalent to about 5 molar equivalents relative to compound (IV-3).

As the base, for example, alkali metal salts such as sodium hydroxide, potassium carbonate and the like; amines such as pyridine, triethylamine and the like; metal hydrides such as sodium hydride and the like; alkali metal alkoxides such as sodium methoxide, potassium t-butoxide and the like; and the like can be mentioned.

The amount of the base to be used is preferably about 1 molar equivalent to about 5 molar equivalents relative to compound (IV-3).

As the reducing agent, for example, metal hydrogen compounds such as diisobutylaluminum hydride and the like; metal hydrogen complex compounds such as sodium cyanoborohydride and the like; and the like can be mentioned.

The amount of the reducing agent to be used is generally 0.1 molar equivalent to 20 molar equivalents relative to compound (IV-3).

The aforementioned reaction using a carbonyl compound can also be carried out without using a reducing agent in the presence of a metal catalyst such as palladium-carbon and the like, and a hydrogen source, in a solvent that does not adversely influence the reaction.

The amount of the metal catalyst to be used is preferably 0.01 molar equivalent to 100 molar equivalents relative to compound (IV-3).

As the hydrogen source, for example, hydrogen gas, formic acid, formic acid amine salt and the like can be mentioned.

As the "solvent that does not adversely influence the reaction" to be used for the alkylation reaction, for example, aromatic hydrocarbons such as toluene and the like; ethers such as tetrahydrofuran and the like; halogenated hydrocarbons such as chloroform and the like; amides such as N,N-dimethylformamide and the like; sulfoxides such as dimethyl sulfoxide and the like; and the like can be mentioned. These solvents may be used in a mixture at an appropriate ratio.

In the alkylation reaction, the reaction temperature is preferably about -10°C to about 100°C.

In the alkylation reaction, the reaction time is generally about 0.5 hr to about 20 hr.

The thus-obtained compound (IV-2) can be isolated and purified by a known separation and purification means, for example, concentration, concentration under reduced pressure, solvent extraction, crystallization, recrystallization, phase transfer, chromatography and the like.

For production of a compound represented by the formula (IV), when the starting material compound has an amino group, a carboxyl group, a hydroxy group or a carbonyl group as a substituent, a protecting group generally used for these groups in the peptide chemistry and the like may be introduced, where the object compound can be obtained by eliminating the protecting group as necessary after the reaction.

As the amino-protecting group, for example, a formyl group, a C₁₋₆ alkyl-carbonyl group, a C₁₋₆ alkoxy-carbonyl group, a benzoyl group, a C₇₋₁₃ aralkyl-carbonyl group, a C₇₋₁₃ aralkyloxy-carbonyl group, a trityl group, a phthaloyl group, an N,N-dimethylaminomethylene group, a silyl group (e.g., trimethylsilyl, triethylsilyl, dimethylphenylsilyl, tert-butyldimethylsilyl, tert-butyldiethylsilyl), a C₂₋₆ alkenyl group and the like can be mentioned. These groups may be optionally substituted by 1 to 3 halogen atoms, a C₁₋₆ alkoxy group, a nitro group and the like.

As the carboxyl-protecting group, for example, a C₁₋₆ alkyl group, a C₇₋₁₃ aralkyl group, a phenyl group, a trityl group, a silyl group (e.g., trimethylsilyl, triethylsilyl, dimethylphenylsilyl, tert-butyldimethylsilyl, tert-butyldiethylsilyl), a C₂₋₆ alkenyl group and the like can be mentioned. These groups may be optionally substituted by 1 to 3 halogen atoms, a C₁₋₆ alkoxy group, a nitro group and the like.

As the hydroxy-protecting group, for example, a C₁₋₆ alkyl group, a phenyl group, a trityl group, a C₇₋₁₃ aralkyl group, a formyl group, a C₁₋₆ alkyl-carbonyl group, a benzoyl group, a C₇₋₁₃ aralkyl-carbonyl group, a 2-tetrahydropyranyl group, a 2-tetrahydrofuranyl group, a silyl group (e.g., trimethylsilyl, triethylsilyl, dimethylphenylsilyl, tert-butyldimethylsilyl, tert-butyldiethylsilyl), a C₂₋₆ alkenyl group (e.g., 1-allyl) and the like can be mentioned. These groups may be optionally substituted by 1 to 3 halogen atoms, a C₁₋₆ alkyl group, a C₁₋₆ alkoxy group, a nitro group and the like.

As the carbonyl-protecting group, for example, cyclic acetal (e.g., 1,3-dioxane), acyclic acetal (e.g., di-C₁₋₆ alkylacetal) and the like can be mentioned.

These protecting groups can be introduced or eliminated according to a method known per se, for example, the method described in Protective Groups in Organic Synthesis, John Wiley and Sons (1980) and the like.

For production of a compound represented by the formula (IV), when the starting material compound can form a salt, the compound may be used as a salt. As such salt, those similar to the salts of a compound represented by the formula (I) can be mentioned.

Of the compounds represented by the formula (IV),
5-(aminomethyl)-2-methyl-4-(4-methylphenyl)-6-neopentylnicotinic acid;
5-(aminomethyl)-6-isobutyl-2-methyl-4-(4-methylphenyl) nicotinic acid;
methyl 3-{[5-(aminomethyl)-6-isobutyl-2-methyl-4-(4-methylphenyl)pyridin-3-yl]methoxy}-1-methyl-1H-pyrazole-4-carboxylate;
{[2-isobutyl-6-methyl-4-(4-methylphenyl)-5-(2-morpholin-4-yl-2-oxoethyl)pyridin-3-yl]methyl}amine;
methyl 3-({[5-(aminomethyl)-6-isobutyl-2-methyl-4-(4-methylphenyl)pyridin-3-yl]acetyl}amino)benzoate;
N-[5-(aminomethyl)-6-isobutyl-2-methyl-4-(4-methylphenyl)pyridin-3-yl]isoxazole-4-carboxamide and the like are particularly preferable.

(6) A compound represented by the formula: 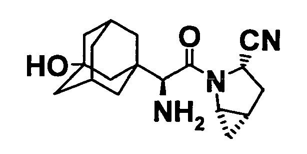 and hydrochloride thereof (BMS-477118, Saxagliptin); TS-021, E-3024, T-6666(TA-6666), 823093, 825964, 815541 and the like.

The agent for increasing a pancreatic insulin content of the present invention (hereinafter sometimes to be abbreviated as the agent of the present invention) can be obtained by combining a blood glucose lowering drug that does not stimulate insulin secretion, and a DPP-IV inhibitor, which are the active ingredients. These active ingredients may be formed into preparations separately or simultaneously together with a pharmacologically acceptable carrier.

Here, various organic or inorganic carrier materials conventionally used as materials for pharmaceutical preparations are used as the pharmacologically acceptable carrier, which are added as excipient, lubricant, binder, disintegrant for solid preparations; and solvent, dissolution aids, suspending agent, isotonicity agent, buffer, soothing agent and the like for liquid preparations. Where necessary, additive for pharmaceutical preparations such as preservative, antioxidant, coloring agent, sweetening agent and the like can be used.

Preferable examples of the excipient include lactose, sucrose, D-mannitol, D-sorbitol, starch, pregelatinized starch, dextrin, crystalline cellulose, low-substituted hydroxypropylcellulose, sodium carboxymethylcellulose, gum arabic, pullulan, light anhydrous silicic acid, synthetic aluminum silicate, magnesium aluminate metasilicate and the like.

Preferable examples of the lubricant include magnesium stearate, calcium stearate, talc, colloidal silica and the like.

Preferable examples of the binder include pregelatinized starch, saccharose, gelatin, gum arabic, methylcellulose, carboxymethylcellulose, sodium carboxymethylcellulose, crystalline cellulose, sucrose, D-mannitol, trehalose, dextrin, pullulan, hydroxypropylcellulose, hydroxypropyl methylcellulose, polyvinylpyrrolidone and the like.

Preferable examples of the disintegrant include lactose, sucrose, starch, carboxymethylcellulose, calcium carboxymethylcellulose, sodium croscarmellose, sodium carboxymethyl starch, light anhydrous silicic acid, low-substituted hydroxypropylcellulose and the like.

Preferable examples of the solvent include water for injection, physiological brine, Ringer's solution, alcohol, propylene glycol, polyethylene glycol, sesame oil, corn oil, olive oil, cottonseed oil and the like.

Preferable examples of the dissolution aids include polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like.

Preferable examples of the suspending agent include surfactants such as stearyltriethanolamine, sodium lauryl sulfate, lauryl aminopropionate, lecithin, benzalkonium chloride, benzethonium chloride, glycerol monostearate and the like; hydrophilic polymers such as polyvinyl alcohol, polyvinylpyrrolidone, sodium carboxymethylcellulose, methylcellulose, hydroxymethylcellulose, hydroxyethylcellulose, hydroxypropylcellulose and the like; polysorbates, polyoxyethylene hydrogenated castor oil; and the like.

Preferable examples of the isotonicity agent include sodium chloride, glycerol, D-mannitol, D-sorbitol, glucose and the like.

Preferable examples of the buffer include phosphate buffer, acetate buffer, carbonate buffer, citrate buffer and the like.

Preferable examples of the soothing agent include benzyl alcohol and the like.

Preferable examples of the preservative include p-oxybenzoates, chlorobutanol, benzyl alcohol, phenethyl alcohol, dehydroacetic acid, sorbic acid and the like.

Preferable examples of the antioxidant include sulfite, ascorbate and the like.

Preferable examples of the coloring agent include watersoluble edible tar pigments (e.g., foodcolors such as Food Color Red Nos. 2 and 3, Food Color Yellow Nos. 4 and 5, Food Color Blue Nos. 1 and 2 and the like); water insoluble lake pigments (e.g., aluminum salt of the aforementioned watersoluble edible tar pigment), natural pigments (e.g., beta carotene, chlorophyll, red iron oxide and yellow ferric oxide) and the like.

Preferable examples of the sweetening agent include saccharin sodium, dipotassium glycyrrhizinate, aspartame, stevia and the like.

The dosage form of the agent of the present invention is, for example, an oral preparation such as tablets (inclusive of sublingual tablets and orally disintegrable tablets), capsules (inclusive of soft capsules and micro capsules), granules, powders, troches, syrups, emulsions, suspensions and the like; or a parenteral preparation such as injections (e.g., subcutaneous injections, intravenous injections, intramuscular injections and intraperitoneal injections), external agents (e.g., preparations for nasal administration, transdermal preparations and ointments), suppositories (e.g., rectal suppositories and vaginal suppositories), pellets, drops, eye drops, pulmonary preparations (inhalations) and the like. In addition, these preparations may be sustained-release preparations (e.g., sustained-release microcapsule), such as an immediate release preparation or a sustained-release preparation. Of these preparations, oral preparations superior in the convenience or compliance are preferable.

The agent of present invention can be produced according to a method conventionally used in the field of pharmaceutical preparation, such as the method described in Japan Pharmacopoeia and the like.

While the content of the active ingredient (blood glucose lowering drug that does not stimulate insulin secretion, and/or DPP-IV inhibitor) in the agent of the present invention varies depending on the kind of the active ingredient, the size of the preparation and the like, it is, for example, 1-90 wt%, preferably 5-80 wt%.

In the agent of the present invention, the mixing ratio of a blood glucose lowering drug that does not stimulate insulin secretion, and a DPP-IV inhibitor can be appropriately determined according to the subject of administration, administration route, target disease, dosage form, combination of pharmaceutical agents and the like. For example, a DPP-IV inhibitor is generally used in an amount of about 0.005-200 parts by weight, preferably about 0.01-100 parts by weight, relative to 1 part by weight of the blood glucose lowering drug that does not stimulate insulin secretion.

The administration mode of the agent of the present invention is not particularly limited, and a blood glucose lowering drug that does not stimulate insulin secretion, and a DPP-IV inhibitor only need to be combined on administration. Such administration mode includes, for example,
(1) administration of a single preparation obtained by simultaneously formulating a blood glucose lowering drug that does not stimulate insulin secretion, and a DPP-IV inhibitor,
(2) simultaneous administration of two kinds of preparations obtained by separately formulating a blood glucose lowering drug that does not stimulate insulin secretion, and a DPP-IV inhibitor, by the same administration route, (3) administration of two kinds of preparations obtained by separately formulating a blood glucose lowering drug that does not stimulate insulin secretion, and a DPP-IV inhibitor, at different times by the same administration route, (4) simultaneous administration of two kinds of preparations obtained by separately formulating a blood glucose lowering drug that does not stimulate insulin secretion, and a DPP-IV inhibitor, by different administration routes, (5) administration of two kinds of preparations obtained by separately formulating a blood glucose lowering drug that does not stimulate insulin secretion, and a DPP-IV inhibitor, at staggered times by different administration routes (for example, administration in the order of a blood glucose lowering drug that does not stimulate insulin secretion and then a DPP-IV inhibitor, or in the reverse order) and the like.

The agent of the present invention can be safely administered orally or parenterally to mammals (e.g., humans, mice, rats, rabbits, dogs, cats, bovines, horses, swines, monkeys).

The dose of the agent of the present invention can be similar to the dose of a blood glucose lowering drug that does not stimulate insulin secretion, or a DPP-IV inhibitor, which are the active ingredients, and can be appropriately determined according to the subject of administration, administration route, target disease, dosage form, combination of pharmaceutical agents and the like.

The dose of the blood glucose lowering drug that does not stimulate insulin secretion, and the DPP-IV inhibitor can be appropriately determined based on their clinical doses.

The dose of the blood glucose lowering drug that does not stimulate insulin secretion is, for example, generally 0.01-500 mg/day, preferably 0.1-100 mg/day, for one adult patient (body weight 60 kg). This amount can be administered in 2 or 3 portions a day.

When an insulin sensitizer is used as a blood glucose lowering drug that does not stimulate insulin secretion, the dose of the insulin sensitizer is generally 0.1-100 mg/day, preferably 1-60 mg/day for one adult patient (body weight 60 kg).

Particularly, when the insulin sensitizer is pioglitazone hydrochloride, the effective amount of pioglitazone hydrochloride is generally 7.5-60 mg/day, preferably 15-45 mg/day, for one adult patient (body weight 60 kg).

When the insulin sensitizer is rosiglitazone maleate, the effective amount of rosiglitazone maleate is generally 1-12 mg/day, preferably 2-8 mg/day, for one adult patient (body weight 60 kg).

When a biguanide (preferably metformin hydrochloride) is used as a blood glucose lowering drug that does not stimulate insulin secretion, the dose of the biguanides is generally 125-2550 mg/day, preferably 250-2550 mg/day for one adult patient (body weight 60 kg).

The dose of the DPP-IV inhibitor is, for example, generally 0.01-1000 mg/day, preferably 0.1-500 mg/day, for one adult patient (body weight 60 kg). This amount can be administered in 2 or 3 portions a day.

The agent of the present invention has an enhanced pancreatic insulin content increasing action as compared to a single administration of a blood glucose lowering drug that does not stimulate insulin secretion, or a DPP-IV inhibitor. In the present specification, the "pancreatic insulin content" means an insulin content of the pancreas.

The "pancreatic insulin content" can be obtained by measuring insulin extracted by a method known per se from a pancreatic tissue of a test animal according to a method known *per se.* The measurement method of insulin may be any as long as insulin can be measured. Specifically, a radioimmunoassay or enzyme immunoassay using one kind of anti-insulin antibody; an enzyme immunoassay using two kinds of anti-insulin antibodies having different epitopes and the like can be used.

In addition, the "pancreatic insulin content" can also be evaluated with pancreatic insulin mRNA or the amount of pancreatic β cells as an index.

Here, the pancreatic insulin mRNA and the amount of pancreatic β cells can be measured by a method known per se. For example, a method by histological staining using an insulin antibody is generally used for the measurement of the amount of pancreatic β cells. In addition, *in situ* hybridization that detects insulin mRNA, a method including labeling with an endogenous or exogenous substance that highly specifically binds to a protein selectively expressed in pancreatic β cells, administering the labeled substance to a test animal, and thereafter measuring the labeling activity and the like may be used. The aforementioned endogenous or exogenous substance can be labeled, for example, with a radioisotope, a luminescence substance (low-molecular-weight compound or protein such as luciferase, GFP and the like), a fluorescent substance and the like.

Moreover, the "pancreatic insulin content" can also be evaluated by a known method used for the assumption of the amount of remaining pancreatic β cells. As such method, for example, a method including a glucagon tolerance test and measurement of activated insulin or C-peptide in the blood and the like can be mentioned. Alternatively, a glucose loading test may be conducted instead of the glucagon tolerance test and then the activated insulin or C-peptide in the blood may be measured. Furthermore, the activated insulin or C-peptide in the blood may be measured without a glucagon tolerance test.

In addition, using a blood glucose lowering drug that does not stimulate insulin secretion, and a DPP-IV inhibitor in combination, the dose of the pharmaceutical agent can be reduced as compared to a single use of each pharmaceutical agent, thereby reducing an unpreferable action of these pharmaceutical agents (e.g., body weight gain action), if they have any.

Moreover, the agent of the present invention has an enhanced activity of lowering plasma glucose level, enhanced activity of lowering glycated hemoglobin level, enhanced activity of increasing insulin level in the blood and the like, as compared to a single administration of a blood glucose lowering drug that does not stimulate insulin secretion, or a DPP-IV inhibitor.

Therefore, the agent of the present invention is useful for the prophylaxis or treatment of diabetes [e.g., type 1 diabetes, type 2 diabetes, type 1.5 (LADA (Latent Autoimmune Diabetes in Adults)), gestational diabetes mellitus, insulin secretion-deficient diabetes, obese diabetes, IGT (Impaired Glucose Tolerance), IFG (Impaired Fasting Glucose), IFG (Impaired Fasting Glycaemia)], diabetic complications [e.g., neuropathy, nephropathy, retinopathy, cataract, macroangiopathy, arteriosclerosis, osteopenia, hyperosmolar diabetic coma, infectious diseases (e.g., respiratory infection, urinary tract infection, gastrointestinal infection, dermal soft tissue infections and inferior limb infection), diabetic gangrene, xerostomia, hypacusis, cerebrovascular disorder and peripheral blood circulation disorder], and the like. Moreover, the agent of the present invention can suppress progression of diabetes into diabetic complications (particularly, diabetic neuropathy, diabetic nephropathy, diabetic retinopathy, arteriosclerosis).

In a mammal having a higher blood glucose level than the normal level, since hyperglycemia itself decreases the pancreatic insulin content, the agent of the present invention can be used for normalization of the blood glucose level in a mammal having a higher blood glucose level than the normal level. Moreover, the agent of the present invention is useful for a mammal showing a low pancreatic (β cell) function to fall into an insulin secretion-deficient condition, from among the mammals having a higher blood glucose level than the normal level.

Furthermore, the agent of the present invention can increase the amount of pancreatic insulin mRNA. The agent of the present invention suppresses pancreatic exhaustion caused by glucose toxicity due to diabetes, lipotoxicity, glycolipotoxicity, oxidant stress, endoplasmic reticulum stress and the like, and can maintain the glucose-dependent insulin secretory ability, which is an important function of pancreatic β cells. In addition, the agent of the present invention can suppress pancreatic β cell death due to diabetes, and promote regeneration or replication of pancreatic β cells. Moreover, the agent of the present invention can promote production of giant pancreatic β cells.

The agent of the present invention can be used in combination with other pharmaceutical agent (hereinafter to be abbreviated as a combination drug), as long as a blood glucose lowering drug that does not stimulate insulin secretion, and a DPP-IV inhibitor, which are active ingredients, are not adversely affected.

Here, as the "combination drug", therapeutic agents for diabetes, therapeutic agents for diabetic complications, therapeutic agents for hyperlipidemia, antihypertensive agents, antiobesity agents, diuretic agents, antithrombotic agents and the like can be mentioned.

The timing of administration of the agent of the present invention and a combination drug is not limited. They may be simultaneously administered to an administration subject or administered in a staggered manner. Moreover, the agent of the present invention and a combination drug may be administered as two kinds of preparations each containing an active ingredient, or may be administered as a single preparation containing both active ingredients.

The dose of the combination drug can be appropriately determined based on the dose clinically employed. The proportion of the agent of the present invention and combination drug can be appropriately determined depending on the administration subject, administration route, target disease, condition, combination and the like. When, for example, the administration subject is human, a combination drug is used in an amount of 0.01-100 parts by weight per 1 part by weight of the active ingredient of the agent of the present invention.

As the aforementioned therapeutic agent for diabetes, for example, insulin preparations (e.g., animal insulin preparations extracted from the pancreas of bovine and swine; human insulin preparations genetically synthesized using *Escherichia coli* or yeast; zinc insulin; protamine zinc insulin; fragment or derivative of insulin (e.g., INS-1), GLP-1 receptor agonists [e.g., GLP-1, NN-2211, AC-2993 (exendin-4), BIM-51077, Aib(8,35)hGLP-1(7,37)NH₂], amylin agonists (e.g., pramlintide), phosphotyrosine phosphatase inhibitors (e.g., sodium vanadate), β3 agonists (e.g., AJ-9677), gluconeogenesis inhibitors (e.g., glycogen phosphorylase inhibitor, glucose-6-phosphatase inhibitor, glucagon antagonist), SGLT (sodium-glucose cotransporter) inhibitors (e.g., T-1095), adiponectin or agonist thereof, IKK inhibitors (e.g., AS-2868), leptin resistance improving drugs, glucokinase activators (e.g., Ro-28-1675), JNK inhibitor, GSK3β inhibitor and the like can be mentioned.

Examples of the therapeutic agent for diabetic complications include aldose reductase inhibitors (e.g., Tolrestat, Epalrestat, Zenarestat, Zopolrestat, Minalrestat, Fidarestat, CT-112, Ranirestat (AS-3201) etc.), neurotrophic factors and increasing drugs thereof (e.g., NGF, NT-3, BDNF, neurotrophin production-secretion promoters described in WO01/14372 (e.g., 4-(4-chlorophenyl)-2-(2-methyl-1-imidazolyl)-5-[3-(2-methylphenoxy)propyl]oxazole etc.)), neuranagenesis stimulators (e.g., Y-128), PKC inhibitors (e.g., ruboxistaurin mesylate), AGE inhibitors (e.g., ALT946, pimagedine, N-phenacylthiazolium bromide (ALT766), EXO-226), reactive oxygen scavengers (e.g., thioctic acid), cerebral vasodilators (e.g., tiapride, mexiletine) and apoptosis signal regulating kinase-1 (ASK-1) inhibitors.

Examples of the therapeutic agent for hyperlipidemia include HMG-CoA reductase inhibitors (e.g., pravastatin, simvastatin, lovastatin, atorvastatin, fluvastatin, itavastatin, rosuvastatin, pitavastatin and salts thereof (e.g., sodium salt, calcium salt), squalene synthase inhibitors (e.g., compounds described in WO97/10224, such as N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid etc.), fibrate compounds (e.g., bezafibrate, clofibrate, simfibrate, clinofibrate), ACAT inhibitors (e.g., Avasimibe, Eflucimibe), anion exchange resins (e.g., colestyramine), probucol, nicotinic acid drugs (e.g., nicomol, niceritrol), ethyl icosapentate, plant sterols (e.g., soysterol, γ-oryzanol) and the like.

Examples of the antihypertensive agent include angiotensin converting enzyme inhibitors (e.g., captopril, enalapril, delapril), angiotensin II antagonists (e.g., candesartan cilexetil, losartan, eprosartan, valsartan, telmisartan, irbesartan, tasosartan, 1-[[2'-(2,5-dihydro-5-oxo-4H-1,2,4-oxadiazol-3-yl)biphenyl-4-yl]methyl]-2-ethoxy-1H-benzimidazole-7-carboxylic acid), calcium antagonists (e.g., manidipine, nifedipine, amlodipine, efonidipine, nicardipine), potassium channel openers (e.g., levcromakalim, L-27152, AL 0671, NIP-121), Clonidine and the like.

Examples of the antiobesity agent include antiobestic agents acting on the central nervous system (e.g., Dexfenfluramine, fenfluramine, phentermine, Sibutramine, amfepramone, dexamphetamine, mazindol, phenylpropanolamine, clobenzorex; MCH receptor antagonists (e.g., SB-568849; SNAP-7941; compounds encompassed in WO01/82925 and WO01/87834); neuropeptide Y antagonists (e.g., CP-422935); cannabinoid receptor antagonists (e.g., SR-141716, SR-147778); ghrelin antagonist; pancreatic lipase inhibitors (e.g., orlistat, ATL-962), β3 agonists (e.g., AJ-9677), peptidic anorexiants (e.g., leptin, CNTF (Ciliary Neurotropic Factor)), cholecystokinin agonists (e.g., lintitript, FPL-15849) and the like.

Examples of the diuretic agent include xanthine derivatives (e.g., sodium salicylate and theobromine, calcium salicylate and theobromine), thiazide preparations (e.g., ethiazide, cyclopenthiazide, trichloromethiazide, hydrochlorothiazide, hydroflumethiazide, benzylhydrochlorothiazide, penflutizide, polythiazide, methyclothiazide), antialdosterone preparations (e.g., spironolactone, triamterene), carbonate dehydratase inhibitors (e.g., acetazolamide), chlorobenzenesulfonamide preparations (e.g., chlortalidone, mefruside, indapamide), azosemide, isosorbide, etacrynic acid, piretanide, bumetanide, furosemide and the like.

Examples of the antithrombotic agent include heparin (e.g., heparin sodium, heparin calcium, dalteparin sodium), warfarin (e.g., warfarin potassium), anti-thrombin drugs (e.g., aragatroban), thrombolytic agents (e.g., urokinase, tisokinase, alteplase, nateplase, monteplase, pamiteplase), platelet aggregation inhibitors (e.g., ticlopidine hydrochloride, cilostazol, ethyl icosapentate, beraprost sodium, sarpogrelate hydrochloride) and the like.

In the agent of the present invention, an enhanced pancreatic insulin content increasing action, an enhanced hypoglycemic action, an enhanced glycated hemoglobin level lowering action, an enhanced blood insulin content increasing action and the like can be respectively obtained even when the DPP-IV inhibitor is replaced by a GLP-1 receptor agonist [e.g., GLP-1, NN-2211, AC-2993 (exendin-4), BIM-51077, Aib(8,35)hGLP-1(7,37)NH₂]. In this case, the GLP-1 receptor agonist can be formed into a preparation as in the case of the DPP-IV inhibitor in the agent of the present invention, and administered to a mammal.

The present invention further relates to an "agent that enhances pancreas protection activity of a DPP-IV inhibitor, which comprises a blood glucose lowering drug that does not stimulate insulin secretion". That is, using a blood glucose lowering drug that does not stimulate insulin secretion, and a DPP-IV inhibitor in combination can potentiate the pancreas protection activity of the DPP-IV inhibitor.

Here, as the blood glucose lowering drug that does not stimulate insulin secretion, and DPP-IV inhibitor, those recited as examples of the aforementioned agent for increasing the pancreatic insulin content can be mentioned, and the dose thereof is the same as that of the aforementioned agent for increasing the pancreatic insulin content.

Examples of the pancreas protection activity include pancreatic insulin content increasing action, pancreatic exhaustion prophylactic/treating action, pancreatic (β cell) function ameliorating effect, pancreatic (β cell) regenerative action, pancreatic (β cell) regeneration promoting action, glucose toxicity suppressing action, lipotoxicity suppressing action, glycolipotoxicity suppressing action, oxidant stress suppressing action, endoplasmic reticulum stress suppressing action, pancreatic β cell apoptosis suppressing action, insulin secretory ability enhancing action and the like. Here, the insulin secretory ability enhancing action can be evaluated by calculating the ratio of a plasma insulin level and a plasma glucose level in an administration subject, where an increase in the "plasma insulin level/plasma glucose level" means enhanced insulin secretory ability. The pancreas protection activity is preferably a pancreatic insulin content increasing action.

The "agent that enhances pancreas protection activity of a DPP-IV inhibitor, which comprises a blood glucose lowering drug that does not stimulate insulin secretion" can be produced using a blood glucose lowering drug that does not stimulate insulin secretion, and in the same manner as in the aforementioned agent for increasing the pancreatic insulin content, and can be used for the prophylaxis or treatment of diabetes, diabetic complications and the like.

In addition, the administration mode of the "agent that enhances pancreas protection activity of a DPP-IV inhibitor, which comprises a blood glucose lowering drug that does not stimulate insulin secretion" may be any of simultaneous administration and administration in a staggered manner of a blood glucose lowering drug that does not stimulate insulin secretion, and a DPP-IV inhibitor, in the same manner as in the aforementioned agent for increasing the pancreatic insulin content.

Furthermore, the "agent that enhances pancreas protection activity of a DPP-IV inhibitor, which comprises a blood glucose lowering drug that does not stimulate insulin secretion" can also be used in combination with the aforementioned combination drug.

The present invention further relates to a "agent that enhances pancreas protection activity of a blood glucose lowering drug that does not stimulate insulin secretion, which comprises a DPP-IV inhibitor". That is, using a DPP-IV inhibitor and a blood glucose lowering drug that does not stimulate insulin secretion in combination can potentiate the pancreas protection activity of the blood glucose lowering drug that does not stimulate insulin secretion.

Here, as the DPP-IV inhibitor, blood glucose lowering drug that does not stimulate insulin secretion, and pancreas protection activity, those similar to the aforementioned can be mentioned, and the dose of the DPP-IV inhibitor and blood glucose lowering drug that does not stimulate insulin secretion is the same as that of the aforementioned agent for increasing the pancreatic insulin content.

The "agent that enhances pancreas protection activity of a blood glucose lowering drug that does not stimulate insulin secretion, which comprises a DPP-IV inhibitor" can be produced using a blood glucose lowering drug that does not stimulate insulin secretion and in the same manner as in the aforementioned agent for increasing the pancreatic insulin content, and can be used for the prophylaxis or treatment of diabetes, diabetic complications and the like.

In addition, the administration mode of the "agent that enhances pancreas protection activity of a blood glucose lowering drug that does not stimulate insulin secretion, which comprises a DPP-IV inhibitor" may be any of simultaneous administration and administration in a staggered manner of a blood glucose lowering drug that does not stimulate insulin secretion, and a DPP-IV inhibitor, in the same manner as in the aforementioned agent for increasing the pancreatic insulin content.

Furthermore, the "agent that enhances pancreas protection activity of a DPP-IV inhibitor, which comprises a DPP-IV inhibitor" can also be used in combination with the aforementioned combination drug.

The present invention further relates to "a method of synergistically protecting the pancreas of a mammal as compared to a single administration of a blood glucose lowering drug that does not stimulate insulin secretion or a DPP-IV inhibitor, which comprises administering a blood glucose lowering drug that does not stimulate insulin secretion, and a DPP-IV inhibitor to the mammal". In other words, using a blood glucose lowering drug that does not stimulate insulin secretion, and a DPP-IV inhibitor in combination, a synergistic pancreas protecting effect can be obtained.

Here, as the blood glucose lowering drug that does not stimulate insulin secretion, DPP-IV inhibitor and mammal, those recited as examples of the aforementioned agent for increasing the pancreatic insulin content can be mentioned, and the dose thereof is the same as that of the aforementioned agent for increasing the pancreatic insulin content.

The aforementioned "synergistic pancreas protecting effect" means a pancreas protecting effect superior to the addition of a "pancreas protecting effect obtained by a single administration of a blood glucose lowering drug that does not stimulate insulin secretion" and a "pancreas protecting effect obtained by a single administration of a DPP-IV inhibitor". In addition, the "pancreas protecting effect" means a pancreatic insulin content increasing effect, a pancreatic exhaustion prophylactic/treating effect, a pancreatic (β cell) function ameliorating effect, a pancreatic (β cell) regenerative effect, a pancreatic (β cell) regeneration promoting effect, a glucose toxicity suppressing effect, lipotoxicity suppressing effect, glycolipotoxicity suppressing effect, oxidant stress suppressing effect, endoplasmic reticulum stress suppressing effect, pancreatic β cell apoptosis suppressing effect, insulin secretory ability enhancing action and the like. The pancreas protecting effect is preferably a pancreatic insulin content increasing effect.

In addition, the administration mode of the blood glucose lowering drug that does not stimulate insulin secretion, and DPP-IV inhibitor is not particularly limited and may be any of simultaneous administration and administration in a staggered manner.

Moreover, the blood glucose lowering drug that does not stimulate insulin secretion, and the DPP-IV inhibitor can also be used in combination with the aforementioned combination drug.

The present invention is explained in more detail by the following Reference Examples, Examples and Experimental Examples. These do not limit the present invention and the present invention can be modified within the range that does not deviate from the scope of the invention.

### Reference Example 1

### Methyl 5-(aminomethyl)-6-isobutyl-2-methyl-4-(4-methylphenyl)nicotinate

1) A suspension of sodium hydride (60% in oil, 8.0 g, 0.2 mol) in tetrahydrofuran (80 mL) was heated under reflux with stirring vigorously. A mixture of methyl isovalerate (11.6 g, 0.1 mol), acetonirtile (10.5 mL, 0.2 mol) and tetrahydrofuran (25 mL) was added dropwise to the obtained suspension over 30 min., and the mixture was heated under reflux for 5 hrs. The reaction mixture was allowed to cool to room temperature, and 2-propanol (5 mL) was added thereto. The mixture was stirred at room temperature for 30 min. The reaction mixture was concentrated under reduced pressure, and the residue was dissolved in water (100 mL) and washed successively with hexane and a mixed solution of hexane-diethyl ether. The aqueous layer was acidified with concentrated hydrochloric acid and extracted with diethyl ether. The extract was washed with water and dried over anhydrous magnesium sulfate. The solvent was evaporated under reduced pressure to give 5-methyl-3-oxohexanenitrile (12.6 g, yield 100%) as a pale yellow oil. The obtained pale yellow oil was used in the next step without further purification.
   ¹H-NMR (CDCl₃) δ:0.96 (6H, d, J = 6.6 Hz), 2.05-2.30 (1H, m), 2.50 (2H, d, J = 7.0 Hz), 3.43 (2H, s).
2) A mixture of 5-methyl-3-oxohexanenitrile (5.0 g, 40 mmol), p-tolualdehyde (4.8 g, 40 mmol), piperidine (0.34 g, 4.0 mmol), acetic acid (0.48 g, 8.0 mmol) and toluene (200 mL) was heated under reflux for 12 hrs. using a Dean-Stark trap. The reaction mixture was allowed to cool to room temperature, washed with saturated brine and dried over anhydrous magnesium sulfate. The solvent was evaporated under reduced pressure and the obtained residue was dissolved in methanol (50 mL). Methyl 3-aminocrotonate (4.6 g, 40 mmol) was added thereto and the mixture was heated under reflux for 6 hrs. The reaction mixture was concentrated under reduced pressure, and the residue was purified by silica gel column chromatography to give methyl 5-cyano-6-isobutyl-2-methyl-4-(4-methylphenyl)-1,4-dihydropyridine-3-carboxylate (7.45 g, yield 57%) as colorless crystals.
   ¹H-NMR (CDCl₃) δ:0.93 (3H, d, J = 6.6 Hz), 0.98 (3H, d, J = 6.6 Hz), 1.80-2.00 (1H, m), 2.10-2.35 (2H, m), 2.30 (3H, s), 2.36 (3H, s), 3.58 (3H, s), 4.57 (1H, s), 5.68 (1H, brs), 7.00-7.20 (4H, m).
3) Methyl 5-cyano-6-isobutyl-2-methyl-4-(4-methylphenyl)-1,4-dihydropyridine-3-carboxylate (7.3 g, 22.5 mmol) was dissolved in 1,4-dioxane (20 mL), and 2N nitric acid (100 mL) was added thereto and the mixture was stirred at 70°C for 1 hr. The reaction mixture was stirred in an ice bath, and ethyl acetate (100 mL) and 2N aqueous sodium hydroxide solution (100 mL) were added thereto. The aqueous layer was separated and extracted with ethyl acetate. The organic layer and the extract were combined, and the combined mixture was washed with saturated brine and dried over anhydrous magnesium sulfate. The solvent was evaporated under reduced pressure and the residue was purified by silica gel column chromatography to give methyl 5-cyano-6-isobutyl-2-methyl-4-(4-methylphenyl)nicotinate (5.94 g, yield 82%) as a white powder.
   ¹H-NMR (CDCl₃) δ:1.01 (6H, d, J = 6.6 Hz), 2.20-2.35 (1H, m), 2.41 (3H, s), 2.63 (3H, s), 2.95 (2H, d, J = 7.4 Hz), 3.60 (3H, s), 7.20-7.30 (4H, m).
4) A mixture of methyl 5-cyano-6-isobutyl-2-methyl-4-(4-methylphenyl)nicotinate (1.00 g, 3.10 mmol), Raney-nickel (4 mL), 25% aqueous ammonia (6 mL), tetrahydrofuran (15 mL) and methanol (45 mL) was stirred in a sealed tube under 0.5 MPa hydrogen atmosphere at room temperature for 6 hrs. The reaction mixture was filtered and the filtrate was concentrated under reduced pressure. The residue was partitioned between ethyl acetate and 10% aqueous potassium carbonate solution. The organic layer was washed with saturated brine and dried over anhydrous magnesium sulfate. The solvent was evaporated under reduced pressure and the residue was purified by silica gel column chromatography to give methyl 5-(aminomethyl)-6-isobutyl-2-methyl-4-(4-methylphenyl)nicotinate (0.97 g, yield 95%) as pale yellow crystals.
   ¹H-NMR (CDCl₃) δ:0.98 (6H, d, J = 6.6 Hz), 1.39 (2H, brs), 2.15-2.30 (1H, m), 2.39 (3H, s), 2.53 (3H, s), 2.80 (2H, d, J = 7.2 Hz), 3.50 (3H, s), 3.66 (2H, s), 7.11 (2H, d, J = 8.0 Hz), 7.21 (2H, d, J = 8.0 Hz).
   melting point: 56-57°C

### Reference Example 2

### 5-{[(tert-butoxycarbonyl)amino]methyl}-6-isobutyl-2-methyl-4-(4-methylphenyl)nicotinic acid

1) To a solution of methyl 5-(aminomethyl)-6-isobutyl-2-methyl-4-(4-methylphenyl)nicotinate (0.90 g, 2.76 mmol) in tetrahydrofuran (25 mL) was added di-t-butyl dicarbonate (0.76 mL, 3.31 mmol), and the mixture was stirred at room temperature for 12 hrs. The reaction mixture was concentrated under reduced pressure, and the residue was purified by silica gel column chromatography to give methyl 5-{[(tert-butoxycarbonyl)amino]methyl}-6-isobutyl-2-methyl-4-(4-methylphenyl)nicotinate (1.16 g, yield 98%) as a white powder.
   ¹H-NMR (CDCl₃) δ:0.97 (6H, d, J = 6.8 Hz), 1.39 (9H, s), 2.10-2.30 (1H, m), 2.39 (3H, s), 2.54 (3H, s), 2.78 (2H, d, J = 7.2 Hz), 3.50 (3H, s), 4.15 (2H, d, J = 4.9 Hz), 4.24 (1H, t, J = 4.9 Hz), 7.06 (2H, d, J = 7.9 Hz), 7.20 (2H, d, J = 7.9 Hz).
2) To a solution of methyl 5-{[(tert-butoxycarbonyl)amino]methyl}-6-isobutyl-2-methyl-4-(4-methylphenyl)nicotinate (1.0 g, 2.34 mmol) in methanol (30 mL) was added 1N aqueous sodium hydroxide solution (10 mL), and the mixture was heated under reflux for 3 days. The reaction mixture was allowed to cool to room temperature, acidified with 0.5N hydrochloric acid and extracted with ethyl acetate. The extract was washed with saturated brine and dried over anhydrous magnesium sulfate. The solvent was evaporated under reduced pressure and the residue was crystallized from water-methanol to give 5-{[(tert-butoxycarbonyl)amino]methyl}-6-isobutyl-2-methyl-4-(4-methylphenyl)nicotinic acid (0.58 g, yield 60%) as a white powder.
   ¹H-NMR (CDCl₃) δ:0.87 (6H, d, J = 6.4 Hz), 1.39 (9H, s), 1.95-2.10 (1H, m), 2.38 (3H, s), 2.67 (3H, s), 2.75 (2H, d, J = 7.2 Hz), 4.13 (2H, d, J = 4.7 Hz), 4.30 (1H, t, J=4.7 Hz), 7.15 (2H, d, J = 7.9 Hz), 7.22 (2H, d, J = 7.9 Hz).

### Reference Example 3

### bis[5-(aminomethyl)-6-isobutyl-2-methyl-4-(4-methylphenyl)nicotinic acid] fumarate

1) A mixed solution of 5-{[(tert-butoxycarbonyl)amino]methyl}-6-isobutyl-2-methyl-4-(4-methylphenyl)nicotinic acid (53.7 g, 130 mmol) and 4N hydrogen chloride - 1,4-dioxane solution (400 mL) was stirred at room temperature for 3 hrs. The precipitated solid was collected by filtration and washed with diisopropyl ether (200 mL). The obtained white solid was dissolved in diisopropyl alcohol (500 mL) and the mixture was stirred at 50°C for 30 min. The obtained mixture was allowed to cool to room temperature, and the mixture was stirred at room temperature for 1 hr. The precipitated solid was collected by filtration and washed with diisopropyl alcohol (50 mL) to give 5-(aminomethyl)-6-isobutyl-2-methyl-4-(4-methylphenyl)nicotinic acid dihydrochloride propan-2-ol solvate (1:1) (46.5 g, yield 80%) as a white solid.
   ¹H-NMR (DMSO-d₆) δ:0.97 (6H, d, J = 6.6 Hz), 1.04 (6H, d, J = 6.0 Hz), 2.16-2.27 (1H, m), 2.37 (3H, s), 2.58 (3H, s), 2.90 (2H, d, J = 7.0 Hz), 3.73-3.86 (3H, m), 7.23 (2H, d, J = 8.1 Hz), 7.30 (2H, d, J = 7.9 Hz), 8.26 (3H, brs).
2) 5-(Aminomethyl)-6-isobutyl-2-methyl-4-(4-methylphenyl)nicotinic acid dihydrochloride propan-2-ol solvate (1:1) (35.6 g, 80 mmol) was suspended in water (80 mL) and 1N aqueous sodium hydroxide solution (160 mL, 160 mmol) was added at room temperature. The mixture was stirred for 1 hr. The precipitated solid was collected by filtration and washed with ethanol (10 mL) to give 5-(aminomethyl)-6-isobutyl-2-methyl-4-(4-methylphenyl)nicotinic acid (13.3 g, yield 53%) (to be sometimes abbreviated to as compound A) as a white solid.
   ¹H-NMR (DMSO-d₆)δ:0.93 (6H, d, J = 6.8 Hz), 2.14-2.25 (1H, m), 2.34 (3H, s), 2.38 (3H, s), 2.70 (2H, d, J = 7.2 Hz), 3.49 (2H, s), 7.14-7.20 (4H, m).
3) 5-(Aminomethyl)-6-isobutyl-2-methyl-4-(4-methylphenyl)nicotinic acid (15.4 g, 49.3 mmol) was suspended in water (400 mL) and the mixture was heated under reflux with stirring for 30 min. Fumaric acid (3.43 g, 29.6 mmol) was added to the obtained suspension and the mixture was stirred at room temperature for 1 hr. The precipitated solid was collected by filtration and washed with water (50 mL) to give bis[5-(aminomethyl)-6-isobutyl-2-methyl-4-(4-methylphenyl)nicotinic acid] fumarate (13.9 g, yield 76%) (to be sometimes abbreviated to as 1/2 fumarate of compound A) as white crystals.
   ¹H-NMR (DMSO-d₆)δ:0.93 (6H, d, J = 6.6 Hz), 2.26-2.28 (1H, m), 2.35 (3H, s), 2.42 (3H, s), 2.72 (2H, d, J = 7.2 Hz), 3.55 (2H, s), 6.49 (1H, s), 7.17 (2H, d, J = 8.3 Hz), 7.21 (2H, d, J = 8.3 Hz).

### Example 1

| | | |
|---|---|---|
| 1) | 1/2 fumarate of compound A | 30 g |
| 2) | pioglitazone hydrochloride | 16.53 g |
| 3) | lactose | 53.47 g |
| 4) | cornstarch | 15 g |
| 5) | calcium carboxymethylcellulose | 44 g |
| 6) | magnesium stearate | 1 g |
| | | 1000 tablets total 160 g |

The entire amounts of 1), 2), 3), 4) and 30 g of 5) are kneaded with water, vacuum dried, and milled. The milled powder is mixed with 14 g of 5) and 1 g of 6), and the mixture is tableted by a tableting machine. In this way, 1000 tablets are obtained.

### Experimental Example 1

Using BKS.Cg-+ Lepr^{db}/+ Lepr^{db}/Jcl mouse (hereinafter to be abbreviated as db/db mouse) (6-week-old, male, Clea Japan, Inc.), which is a diabetes model with lowered pancreatic insulin content, and BKS.Cg-m +/+ Lepr^{db}/Jcl mouse (hereinafter to be abbreviated as db/+m mouse) (6-week-old, male, Clea Japan, Inc.), which is a normal model, a plasma glucose level lowering effect, a pancreatic insulin content increasing effect and a plasma insulin/plasma glucose value increasing effect, afforded by a combination of a blood glucose lowering drug that does not stimulate insulin secretion, and a DPP-IV inhibitor, was studied.

First of all, db/db mice (32 mice) were divided into 4 groups of A to D (8 mice in each group), and a powder diet (trade name: CE-2, Clea Japan, Inc., hereinafter the same) was given to group A (control group), a powder diet containing pioglitazone hydrochloride (0.01(w/w)% as pioglitazone) was given to group B, a powder diet containing 1/2 fumarate of compound A (0.03(w/w)% as compound A) was given to group C, and a powder diet containing pioglitazone hydrochloride (0.01(w/w)% as pioglitazone) and 1/2 fumarate of compound A (0.03(w/w)% as compound A) was given to group D, for 3 consecutive weeks.

In addition, a powder diet was given to db/+m mice for 3 consecutive weeks, and the mice were used as group E (normal group).

No significant difference was observed in the feed intake of the mice in the above-mentioned groups A to E.

In the above-mentioned groups A to E, blood samples were collected from mice on day 21 from the start of the powder diet administration, and plasma glucose level and plasma insulin level were measured. On day 23 from the start of the powder diet administration, the pancreas was removed from the mice, and the pancreatic insulin content was measured.

The plasma glucose level was measured by an enzyme method using L type Wako Glu2 (trade name, Wako Pure Chemical Industries, Ltd.).

In addition, the plasma insulin level was measured by a radioimmunoassay (trade name, ShionoRIA insulin), and the plasma insulin level was divided by the plasma glucose level to determine the plasma insulin/plasma glucose value.

The pancreatic insulin content was measured as follows.

First, the mouse pancreas was disrupted in its 10 times weight of 75% ethanol (containing 0.15 M hydrochloric acid). The obtained disrupted solution was left standing overnight under the shading conditions at 4·^{□}C, and centrifuged at 15000 rpm for 5 min. The obtained supernatant was appropriately diluted with PBS(-) (phosphate-buffered saline) containing 0.1% BSA (bovine serum albumin). The insulin content of the diluted solution was measured in the same manner as in the above-mentioned plasma insulin level, and the insulin content per pancreatic tissue weight was calculated.

The results are shown in [Table 1] to [Table 4]. The values in the tables show mean (n=8) ± standard deviation.

**[Table 1] plasma glucose level (mg/dl)**

| group | plasma glucose level |
|---|---|
| group A (control) | 504.6±42.5 |
| group B (pioglitazone) | 411.0±54.4 |
| group C (compound A) | 502.0±54.3 |
| group D (pioglitazone+compound A) | 267.1±89.7 |
| group E (normal group) | 146.4±20.1 |

**[Table 2] pancreatic insulin content (µU/mg tissue)**

| group | pancreatic insulin content |
|---|---|
| group A (control) | 911.7±420.3 |
| group B (pioglitazone) | 2314.2±1285.4 |
| group C (compound A) | 918.4± 328.6 |
| group D (pioglitazone+compound A) | 4962.7±2441.0 |
| group E (normal group) | 4823.6±754.9 |

**[Table 3] plasma insulin level (µU/mL)**

| group | plasma insulin level |
|---|---|
| group A (control) | 97.8±53.8 |
| group B (pioglitazone) | 289.2±261.8 |
| group C (compound A) | 135.5±115.4 |
| group D (pioglitazone+compound A) | 538.2±363.6 |
| group E (normal group) | 55.6±28.1 |

**[Table 4] plasma insulin/plasma glucose value (µU/mg)**

| group | plasmainsulin/plasma glucose value |
|---|---|
| group A (control) | 20.1±12.3 |
| group B (pioglitazone) | 72.9±64.5 |
| group C (compound A) | 29.0±28.4 |
| group D (pioglitazone+compound A) | 248.9±214.0 |
| group E (normal group) | 37.4±15.9 |

As shown in [Table 1]-[Table 4], the combination of a blood glucose lowering drug that does not stimulate insulin secretion, and a DPP-IV inhibitor afforded a synergistic lowering effect on the plasma glucose level (interaction P=0.0036), a synergistic increasing effect on the pancreatic insulin content (interaction P=0.0128), and a synergistic increasing effect on the plasma insulin/plasma glucose value (interaction P=0.0455).

### Experimental Example 2

Using db/db mouse (6-week-old, male, Clea Japan, Inc.) and db/+m mouse (6-week-old, male, Clea Japan, Inc.), a plasma glucose level lowering effect, a pancreatic insulin content increasing effect and a plasma insulin/plasma glucose value increasing effect, afforded by a combination of a blood glucose lowering drug that does not stimulate insulin secretion, and a DPP-IV inhibitor, was studied.

First of all, db/db mice (32 mice) were divided into 4 groups of F to I (8 mice in each group), and a powder diet (trade name: CE-2, Clea Japan, Inc., hereinafter the same) was given to group F (control group), a powder diet containing pioglitazone hydrochloride (0.01(w/w)% as pioglitazone) was given to group G, a powder diet containing vildagliptin (0.03(w/w)%) was given to group H, and a powder diet containing pioglitazone hydrochloride (0.01(w/w)% as pioglitazone) and vildagliptin (0.03(w/w)%) was given to group I, for 5 consecutive weeks.

In addition, a powder diet was given to db/+m mice for 5 consecutive weeks, and the mice were used as group J (normal group) .

No significant difference was observed in the feed intake of the mice in the above-mentioned groups F to I.

In the above-mentioned groups F to I, blood samples were collected from mice on day 35 from the start of the powder diet administration, and plasma glucose level and plasma insulin level were measured. On day 37 from the start of the powder diet administration, the pancreas was removed from the mice, and the pancreatic insulin content was measured.

The plasma glucose level, plasma insulin level, plasma insulin/plasma glucose value, and the pancreatic insulin content were measured in the same manner as in Experimental Example 1.

The results are shown in [Table 5] to [Table 8]. The values in the tables show mean (n=8) ± standard deviation.

**[Table 5] plasma glucose level (mg/dl)**

| group | plasma glucose level |
|---|---|
| group F (control) | 484.1±65.7 |
| group G (pioglitazone) | 348.5±97.4 |
| group H (vildagliptin) | 520.8±43.5 |
| group I (pioglitazone+vildagliptin) | 292.3±78.0 |
| group J (normal group) | 127.6±14.8 |

**[Table 6] pancreatic insulin content (µU/mg tissue)**

| group | pancreatic insulin content |
|---|---|
| group F (control) | 500.3±127.5 |
| group G (pioglitazone) | 834.7±275.3 |
| group H (vildagliptin) | 559.2±169.2 |
| group I (pioglitazone+vildagliptin) | 1472.7±661.0 |
| group J (normal group) | 3840.8±638.0 |

**[Table 7] plasma insulin level (µU/mL)**

| group | plasma insulin level |
|---|---|
| group F (control) | 77.2±34.8 |
| group G (pioglitazone) | 200.2±78.0 |
| group H (vildagliptin) | 80.2±44.5 |
| group I (pioglitazone+vildagliptin) | 573.3±381.3 |
| group J (normal group) | 22.4±24.2 |

**[Table 8] plasma insulin/plasma glucose value (µU/mg)**

| group | plasma insulin/plasma glucose value |
|---|---|
| group F (control) | 16.3±8.3 |
| group G (pioglitazone) | 63.9±35.9 |
| group H (vildagliptin) | 15.1±7.1 |
| group I (pioglitazone+vildagliptin) | 243.7±206.3 |
| group J (normal group) | 18.1±21.0 |

As shown in [Table 5]-[Table 8], the combination of a blood glucose lowering drug that does not stimulate insulin secretion, and a DPP-IV inhibitor afforded a synergistic increasing effect on the pancreatic insulin content (interaction P=0.0367), and a synergistic increasing effect on the plasma insulin/plasma glucose value (interaction P=0.0212).

### Experimental Example 3

Using db/db mouse (6-week-old, male, Clea Japan, Inc.) and db/+m mouse (6-week-old, male, Clea Japan, Inc.), a plasma glucose level lowering effect, a pancreatic insulin content increasing effect and a plasma insulin/plasma glucose value increasing effect, afforded by a combination of a blood glucose lowering drug that does not stimulate insulin secretion, and a DPP-IV inhibitor, was studied.

First of all, db/db mice (32 mice) were divided into 4 groups of K to N (8 mice in each group), and a powder diet (trade name: CE-2, Clea Japan, Inc., hereinafter the same) was given to group K (control group), a powder diet containing pioglitazone hydrochloride (0.01(w/w)% as pioglitazone) was given to group L, a powder diet containing sitagliptin hydrochloride (0.03(w/w)% as sitagliptin) was given to group M, and a powder diet containing pioglitazone hydrochloride (0.01(w/w)% as pioglitazone) and sitagliptin hydrochloride (0.03(w/w)% as sitagliptin) was given to group N, for 4 consecutive weeks.

In addition, a powder diet was given to db/+m mice for 4 consecutive weeks, and the mice were used as group O (normal group).

No significant difference was observed in the feed intake of the mice in the above-mentioned groups K to N.

In the above-mentioned groups K to N, blood samples were collected from mice on day 28 from the start of the powder diet administration, and plasma glucose level and plasma insulin level were measured. On day 29 from the start of the powder diet administration, the pancreas was removed from the mice, and the pancreatic insulin content was measured.

The plasma glucose level, plasma insulin level, plasma insulin/plasma glucose value, and the pancreatic insulin content were measured in the same manner as in Experimental Example 1.

The results are shown in [Table 9] to [Table 12]. The values in the tables show mean (n=8) ± standard deviation.

**[Table 9] plasma glucose level (mg/dl)**

| group | plasma glucose level |
|---|---|
| group K (control) | 533.8±82.8 |
| group L (pioglitazone) | 341.0±100.6 |
| group M (sitagliptin) | 492.3±38.3 |
| group N (pioglitazone+sitagliptin) | 146.6±64.9 |
| group O (normal group) | 178.5±13.4 |

**[Table 10] pancreatic insulin content (µU/mg tissue)**

| group | pancreatic insulin content |
|---|---|
| group K (control) | 387.4±105.0 |
| group L (pioglitazone) | 1117.2±479.7 |
| group M (sitagliptin) | 331.1±79.6 |
| group N (pioglitazone+sitagliptin) | 4100.5±1957.6 |
| group O (normal group) | 2898.3±219.8 |

**[Table 11] plasma insulin level (µU/mL)**

| group | plasma insulin level |
|---|---|
| group K (control) | 146.6±55.9 |
| group L (pioglitazone) | 739.5±472.8 |
| group M (sitagliptin) | 103.1±47.2 |
| group N (pioglitazone+sitagliptin) | 694.5±316.0 |
| group O (normal group) | 75.8±64.1 |

**[Table 12] plasma insulin/plasma glucose value (µU/mg)**

| group | plasma insulin/plasma glucose value |
|---|---|
| group K (control) | 28.9±13.7 |
| group L (pioglitazone) | 261.7±217.9 |
| group M (sitagliptin) | 21.6±11.3 |
| group N (pioglitazone+sitagliptin) | 564.1±328.7 |
| group O (normal group) | 40.5±31.2 |

As shown in [Table 9]-[Table 12], the combination of a blood glucose lowering drug that does not stimulate insulin secretion, and a DPP-IV inhibitor afforded a synergistic lowering effect on plasma glucose level (interaction P=0.0077), a synergistic increasing effect on the pancreatic insulin content (interaction P=0.0002), and a synergistic increasing effect on the plasma insulin/plasma glucose value (interaction P=0.0348).

### Industrial Applicability

The agent of the present invention for increasing a pancreatic insulin content provides a superior pancreatic insulin content increasing effect and is useful for the treatment of diabetes and the like.

This application is based on a patent application No. 2004-246620 filed in Japan, the contents of which are all hereby incorporated.

## Claims

1. An agent for increasing a pancreatic insulin content, which comprises a blood glucose lowering drug that does not stimulate insulin secretion, and a dipeptidyl-peptidase IV inhibitor in combination.

2. The agent of claim 1, wherein the blood glucose lowering drug that does not stimulate insulin secretion is an insulin sensitizer.

3. The agent of claim 2, wherein the insulin sensitizer is pioglitazone or a salt thereof.

4. A method of increasing a pancreatic insulin content of a mammal, which comprises administering a blood glucose lowering drug that does not stimulate insulin secretion, and a dipeptidyl-peptidase IV inhibitor to the mammal.

5. Use of a blood glucose lowering drug that does not stimulate insulin secretion, and a dipeptidyl-peptidase IV inhibitor for the production of an agent for increasing a pancreatic insulin content.

6. An agent that enhances pancreas protection activity of a dipeptidyl-peptidase IV inhibitor, which comprises a blood glucose lowering drug that does not stimulate insulin secretion.

7. The agent of claim 6, wherein the blood glucose lowering drug that does not stimulate insulin secretion is an insulin sensitizer.

8. The agent of claim 7, wherein the insulin sensitizer is pioglitazone or a salt thereof.

9. A method of enhancing a pancreas protection activity of a dipeptidyl-peptidase IV inhibitor in a mammal, which comprises administering a blood glucose lowering drug that does not stimulate insulin secretion to the mammal.

10. Use of a blood glucose lowering drug that does not stimulate insulin secretion for the production of an agent that enhances pancreas protection activity of a dipeptidyl-peptidase IV inhibitor.

11. An agent that enhances pancreas protection activity of a blood glucose lowering drug that does not stimulate insulin secretion, which comprises a dipeptidyl-peptidase IV inhibitor.

12. The agent of claim 11, wherein the blood glucose lowering drug that does not stimulate insulin secretion is an insulin sensitizer.

13. The agent of claim 12, wherein the insulin sensitizer is pioglitazone or a salt thereof.

14. A method of enhancing a pancreas protection activity of a blood glucose lowering drug that does not stimulate insulin secretion in a mammal, which comprises administering a dipeptidyl-peptidase IV inhibitor to the mammal.

15. Use of a dipeptidyl-peptidase IV inhibitor for the production of an agent that enhances pancreas protection activity of a blood glucose lowering drug that does not stimulate insulin secretion.

16. A method of synergistically protecting the pancreas of a mammal as compared to a single administration of a blood glucose lowering drug that does not stimulate insulin secretion, or a dipeptidyl-peptidase IV inhibitor, which comprises administering a blood glucose lowering drug that does not stimulate insulin secretion, and a dipeptidyl-peptidase IV inhibitor to the mammal.
